# EUROPEAN PATENT APPLICATION

(11) **EP 3 875 958 A1**
(43) Date of publication of application: **08.09.2021**
(21) Application number: 19880081.5
(22) Date of filing: 25.10.2019
(51) Int. Cl.: G01N 33/53

(54) **METHOD FOR OBTAINING INFORMATION ON RISK OF REDUCED RESPIRATORY FUNCTION IN PATIENT WITH INTERSTITIAL PNEUMONIA, AND USE THEREOF**

(30) Priority: 29.10.2018 JP 2018202996
(71) Applicant: National University Corporation Tokyo Medical and Dental University, Tokyo 113-8510 (JP); SYSMEX CORPORATION, Kobe-shi Hyogo 651-0073 (JP)
(72) Inventor: MIYAZAKI, Yasunari, Tokyo 113-8510 (JP); NUKUI, Yoshihisa, Tokyo 113-8510 (JP); HASEGAWA, Takehiro, Kobe-shi, Hyogo 651-0073 (JP)
(74) Representative: De Clercq & Partners
(86) International application number: PCT/JP2019/042029
(87) International publication number: WO 2020/090683

(57) **Abstract**

The present invention relates to a method for obtaining information on a risk of a decrease in a respiratory function of a patient with interstitial pneumonia. The present invention relates to method for determining a risk of a decrease in a respiratory function of a patient with interstitial pneumonia. The present invention relates to a device and a computer program for determining a risk of a decrease in a respiratory function of a patient with interstitial pneumonia.

## Description

### TECHNICAL FIELD

The present invention relates to a method for obtaining information on a risk of a decrease in a respiratory function of a patient with interstitial pneumonia. The present invention relates to method for determining a risk of a decrease in a respiratory function of a patient with interstitial pneumonia. The present invention relates to a device and a computer program for determining a risk of a decrease in a respiratory function of a patient with interstitial pneumonia.

### BACKGROUND ART

Interstitial pneumonia is a disease in which inflammation and fibrogenesis occur in an interstitial tissue of a lung, especially an alveolar septum. In the early stage of interstitial pneumonia, there are often few symptoms. However, as interstitial pneumonia progresses, fibrogenesis of a lung decreases vital capacity and gas exchange capacity to appear a symptom of dyspnea. The causes of interstitial pneumonia are diverse. When the cause is allergy caused by inhalation of a specific antigen, it is called hypersensitivity pneumonitis (HP), and when the cause is unknown, it is called idiopathic interstitial pneumonia (IIPs). In IIPs, the causative antigen is unknown, and the pathological mechanism is the same as HP.

Treatment of interstitial pneumonia differs between HP and IIPs. Antigen avoidance is mainly performed in the treatment of HP, and a steroid and an immunosuppressive drug are used for inflammation. In the treatment of idiopathic pulmonary fibrosis (IPF), which accounts for the majority of IIPs, anti-inflammatory therapy is rather not performed, but antifibrotic drugs such as pirfenidone are used. The effectiveness of a treatment is usually evaluated by respiratory functions such as vital capacity 6 months after the start of the treatment.

### PRIOR ART DOCUMENTS

### NON-PATENT DOCUMENTS

Non-Patent Document 1: Yasunari Miyazaki, Study on chronic and fibrogenesis pathology in hypersensitivity pneumonia, The Japanese Journal of Sarcoidosis and Other Granulomatous Disorders, 2014, vol. 34, p. 11-17
Non-Patent Document 2: Kishi M. et al., Pathogenesis of cBFL in common with IPF? Correlation of IP-10/TARC ratio with histological patterns, Thorax, 2008, vol. 63, p. 810-816

### DISCLOSURE OF THE INVENTION

### PROBLEMS TO BE SOLVED BY THE INVENTION

As mentioned above, the effectiveness of a treatment for interstitial pneumonia is evaluated by the respiratory function 6 months after the start of treatment, so when the risk of a decrease in a respiratory function is known, the treatment effect can be determined in advance. Therefore, the determination of the risk of a decrease in a respiratory function is useful for deciding a treatment policy. On the other hand, the current treatment of interstitial pneumonia only suppresses the progression of the disease and does not restore the respiratory function decreased due to damage and fibrogenesis of a lung. When the risk of a decrease in a respiratory function can be determined, it becomes possible to actively try a treatment for a high-risk patient before the progression of interstitial pneumonia. However, a method for determining the risk of future decrease in a respiratory function of a patient, or a biomarker that correlates with such a risk are not known.

Chemokine is known as one of the biomarkers associated with interstitial pneumonia. For example, Non-Patent Document 1 discloses that CCL17 (CC chemokine ligand 17) can be a marker for determining the risk of developing an acute exacerbation. However, the acute exacerbation is the rapid deterioration of the symptoms of interstitial pneumonia within a few days, resulting in fatal dyspnea. That is, the acute exacerbation differs from conditions in which respiratory function is decreased by the normal progression of interstitial pneumonia. Non-Patent Document 2 discloses an increase in CCL17 concentration in the serum of a patient with usual interstitial pneumonia (UIP). However, Non-Patent Document 2 does not disclose the relativity between chemokine and the risk of a decrease in a respiratory function of a patient with interstitial pneumonia.

In view of the above circumstances, an object of the present invention is to provide a means capable of determining the risk of a decrease in a respiratory function of a patient with interstitial pneumonia.

### SOLUTIONS TO THE PROBLEMS

The present inventors investigated the relativity between the chemokine concentration in a blood sample of a patient with interstitial pneumonia and the respiratory function of each patient. As a result, the present inventors found out that there was a significant correlation between the concentrations of CCL17, CXCL9 (CXC chemokine ligand 9), and CXCL11 (CXC chemokine ligand 11), and the subsequent decrease in a respiratory function of the patient, and completed the present invention.

Therefore, the present invention provides a method for obtaining information on a risk of a decrease in a respiratory function of a patient with interstitial pneumonia, the method including measuring at least one biomarker in a biological sample of the patient with interstitial pneumonia, wherein the biomarker includes CCL17, CXCL9, and CXCL11, and a measurement result of the biomarker is an index of the risk of a decrease in the respiratory function of the patient.

The present invention provides a method for determining a risk of a decrease in a respiratory function of a patient with interstitial pneumonia, the method including measuring at least one biomarker in a biological sample of the patient with interstitial pneumonia, and determining the risk of a decrease in the respiratory function of the patient based on a measurement result of the biomarker, the biomarkers including CCL17, CXCL9, and CXCL11.

The present invention provides a reagent kit for use in the above method, the reagent kit including a reagent containing a substance that can specifically bind to CCL17 and/or a reagent containing a substance that can specifically bind to CXCL9 and/or a reagent containing a substance that can specifically bind to CXCL11.

The present invention provides a device for determining a risk of a decrease in a respiratory function of a patient with interstitial pneumonia, the device including a computer including a processor and a memory under the control of the processor, wherein the memory records a computer program for the computer to execute a step of obtaining a measurement result of at least one biomarker in a biological sample of the patient with interstitial pneumonia; and a step of determining the risk of a decrease in a respiratory function of the patient based on the measurement result of the biomarker, wherein the biomarker includes CCL17, CXCL9, and CXCL11.

The present invention provides a computer program for determining a risk of a decrease in a respiratory function of a patient with interstitial pneumonia, wherein the computer program is recorded on a medium that can be read by a computer and is recorded for the computer to execute a step of obtaining a measurement result of at least one biomarker in a biological sample of the patient with interstitial pneumonia; and a step of determining the risk of a decrease in a respiratory function of the patient based on the measurement result of the biomarker, wherein the biomarker includes CCL17, CXCL9, and CXCL11.

### EFFECTS OF THE INVENTION

According to the present invention, it is possible to obtain information on a risk of a decrease in a respiratory function of a patient with interstitial pneumonia.

### BRIEF DESCRIPTION OF THE DRAWINGS

Fig. 1A is a diagram showing an example of the appearance of a reagent kit of the present invention.
Fig. 1B is a diagram showing an example of the appearance of a reagent kit of the present invention.
Fig. 2 is a schematic view showing an example of a device for determining a risk of a decrease in a respiratory function.
Fig. 3 is a block diagram showing a hardware configuration of a device for determining a risk of a decrease in a respiratory function.
Fig. 4A is a flowchart of determination using a device for determining a risk of a decrease in a respiratory function.
Fig. 4B is a flowchart of determination using a device for determining a risk of a decrease in a respiratory function.
Fig. 4C is a flowchart of determination using a device for determining a risk of a decrease in a respiratory function.
Fig. 4D is a flowchart of determination using a device for determining a risk of a decrease in a respiratory function.
Fig. 4E is a flowchart of determination using a device for determining a risk of a decrease in a respiratory function.
Fig. 4F is a flowchart of determination using a device for determining a risk of a decrease in a respiratory function.
Fig. 4G is a flowchart of determination using a device for determining a risk of a decrease in a respiratory function.
Fig. 5 is a diagram showing a result of classifying patients with interstitial pneumonia based on concentrations of CCL17 and CXCL9 in the serum by a cluster analysis.
Fig. 6 is a graph showing the amount of change in vital capacity of patients in the G1 group, G2 group, and G3 group after 6 months.
Fig. 7A is a graph showing the decreasing rate (%) of vital capacity after 6 months in the high value group (High) and the low value group (Low) divided by the median of a serum CCL17 concentration.
Fig. 7B is a graph showing the decreasing rate (%) of vital capacity after 6 months in the high value group (High) and the low value group (Low) divided by the median of a serum CXCL9 concentration.
Fig. 7C is a graph showing the decreasing rate (%) of vital capacity after 6 months in the high value group (High) and the low value group (Low) divided by the median of a ratio of the serum CCL17 concentration to the serum CXCL9 concentration.
Fig. 7D is a graph showing the decreasing rate (%) of vital capacity after 6 months in the high value group (HIGH) and low value group (LOW) divided by the median of a serum CXCL11 concentration.
Fig. 7E is a graph showing the decreasing rate (%) of vital capacity after 6 months in the high value group (HIGH) and low value group (LOW) divided by the median of a ratio of the serum CCL17 concentration to a serum CXCL10 concentration.
Fig. 7F is a graph showing the decreasing rate (%) of vital capacity after 6 months in the high value group (HIGH) and low value group (LOW) divided by the median of a ratio of the serum CCL17 concentration to the serum CXCL11 concentration.
Fig. 7G is a graph showing the decreasing rate (%) of vital capacity after 6 months in the high value group (HIGH) and low value group (LOW) divided by the median of a ratio of a serum CCL22 concentration to the serum CXCL11 concentration.
Fig. 7H is a graph showing the decreasing rate (%) of vital capacity after 6 months in the high value group (HIGH) and low value group (LOW) groups divided by the median ratio of the serum CCL22 concentration to the serum CXCL9 concentration.
Fig. 8A is an ROC (receiver operating characteristic) curve when the risk of a decrease in a respiratory function was determined based on the serum CCL17 concentration.
Fig. 8B is an ROC curve when the risk of a decrease in a respiratory function was determined based on the serum CXCL9 concentration.
Fig. 8C is an ROC curve when the risk of a decrease in a respiratory function was determined based on the ratio of the serum CCL17 concentration to the serum CXCL9 concentration.
Fig. 8D is an ROC curve when the risk of a decrease in a respiratory function was determined based on the serum CXCL11 concentration.
Fig. 8E is an ROC curve when the risk of a decrease in a respiratory function was determined based on the ratio of the serum CCL17 concentration to the serum CXCL10 concentration.
Fig. 8F is an ROC curve when the risk of a decrease in a respiratory function was determined based on the ratio of the serum CCL17 concentration to the serum CXCL11 concentration.
Fig. 8G is an ROC curve when the risk of a decrease in a respiratory function was determined based on the ratio of the serum CCL22 concentration to the serum CXCL9 concentration.
Fig. 8H is an ROC curve when the risk of a decrease in a respiratory function was determined based on the ratio of the serum CCL22 concentration to the serum CXCL11 concentration.

### MODE FOR CARRYING OUT THE INVENTION

### [1. Method for obtaining information about risk of decrease in respiratory function]

In the method for obtaining information on a risk of a decrease in a respiratory function of the present invention (hereinafter, also simply referred to as a "method"), at least one biomarker in a biological sample of a patient with interstitial pneumonia is measured.

A subject in the method of the present invention is not particularly limited as long as the patient is a patient diagnosed with interstitial pneumonia but is preferably an untreated patient with interstitial pneumonia. Alternatively, the subject may be a person suspected of having interstitial pneumonia. As used herein, the term "patient with interstitial pneumonia" includes both a patient diagnosed with interstitial pneumonia and a person suspected of having interstitial pneumonia. The kind of interstitial pneumonia is not particularly limited but is preferably hypersensitivity pneumonia.

The biological sample is not particularly limited as long as it is a sample containing the biomarker described below. Examples of such a biological sample include a blood sample, and a bronchoalveolar lavage fluid. Examples of the blood sample include blood (whole blood) collected from a subject and plasma or serum prepared from the blood. In the present embodiment, the serum is particularly preferable.

When the biological sample contains insoluble contaminants such as a cell, the contaminants may be removed from the biological sample by known means such as centrifugation and filtration. Furthermore, the biological sample may be diluted with an appropriate aqueous medium, as necessary. Such an aqueous medium is not particularly limited as long as it does not interfere with the measurement described below, and examples of the aqueous medium include water, physiological saline, and a buffer. The buffer is not particularly limited as long as it has a buffering action at a pH near neutral (for example, a pH of 6 or more and 8 or less). Examples of such a buffer include Good buffers such as HEPES, MES, and PIPES, Tris buffered saline (TBS), and phosphate buffered saline (PBS).

Examples of the biomarker measured in the method of the present invention include CCL17, CXCL9, and CXCL11. CCL17 is a kind of Th2 type chemokines and is also called TARC (Thymus- and activation-regulated chemokine). CXCL9 is a kind of Th1 type chemokines and is also called MIG (Monokine induced by interferony). CCL17 is a ligand for a CCR4 receptor expressed on the Th2 cell surface, and CXCL9 is a ligand for a CXCR3 receptor expressed on the Th1 cell surface. CXCL11 is a chemokine and also called I-TAC (Interferon-inducible T-cell Chemoattractant) and is the ligand for the CXCR3 receptor like CXCL9. The amino acid sequences of CCL17, CXCL9 and CXCL11 themselves are known and can be known from known databases such as NCBI (National Center for Biotechnology Information).

In the present embodiment, the biomarker may further include at least one selected from a group consisting of CCL22 and CXCL10. CCL22 is a chemokine, also called MDC (Macrophage-derived chemokine), and is the ligand for the CCR4 receptor like CCL17. It is known that both the CCL17 and CCL22 genes are present on chromosome 16 in a human. CXCL10 is a chemokine and also called IP-10 (Interferon-inducible Protein-10) and is the ligand for the CXCR3 receptor like CXCL9. It is known that CXCL9, CXCL10, and CXCL11 are all produced and induced from various cells by interferon gamma stimulation. The amino acid sequences of CCL22 and CXCL10 themselves are known and can be known from known databases such as NCBI.

As used herein, "measuring a biomarker" includes determining a value of the amount or concentration of the biomarker and obtaining information reflecting the amount or concentration of the biomarker. "Information reflecting the amount or concentration of the biomarker" means an index that changes according to the amount or concentration of the biomarker in a biological sample or a measurement sample prepared from the biological sample. Such an index is preferably index of an optical change that is visible or mechanically measurable. Examples of the index of the optical change include the emission intensity, fluorescence intensity, absorbance, turbidity, and coloring intensity.

The information reflecting the amount or concentration of the biomarker may be shown qualitatively, quantitatively, or semi-quantitatively. The information qualitatively shown is information indicating the presence or absence of the biomarker. The information shown quantitatively is numerical information such as a numerical value obtained by a measuring device (hereinafter, also referred to as "raw data") and a value calculated from the numerical value. Examples of the value calculated from the raw data include a value obtained by subtracting a value of the negative control sample or a background value from the raw data. Based on the quantitative information, the value of the amount or concentration of the biomarker can be determined. The information shown semi-quantitatively is information that indicates the amount or concentration of the biomarker in a stepwise way by a word, a number (indicating a class), color, and the like. For example, words such as "below a detection limit", "little", "medium", and "much" may be used.

In the present invention, the measurement result of the biomarker includes a value, information, and a combination of them obtained by measuring the biomarker. In a preferred embodiment, the measurement result of the biomarker is quantitative information reflecting the amount or concentration of the biomarker and/or a value of the amount or concentration of the biomarker determined based on the quantitative information. Hereinafter, the quantitative information reflecting the amount or concentration of the biomarker and/or the value of the amount or concentration of the biomarker is also referred to as "measured value of the biomarker". Examples of the measured value of the biomarker include a measured value of CCL17, a measured value of CXCL9, a measured value of CCL22, a measured value of CXCL10, and a measured value of CXCL11.

The measurement result of the biomarker may be a measured value of one biomarker or may be a value calculated by using measured values of two or more biomarkers. An arbitrary coefficient and/or constant may be further used in the calculation, as necessary. For example, a value calculated using the measured values of two biomarkers include the ratio, product, sum, and difference of the two measured values. In the present invention, the ratio of the measured value of CCL17 to the measured value of CXCL9 (hereinafter, also referred to as "CCL17/CXCL9"), the ratio of the measured value of CXCL9 to the measured value of CCL17 (hereinafter, also referred to as "CXCL9/CCL17"), the ratio of the measured value of CCL17 to the measured value of CXCL10 (hereinafter, also referred to as "CCL17/CXCL10"), the ratio of the measured value of CXCL10 to the measured value of CCL17 (hereinafter, also referred to as "CXCL10/CCL17"), the ratio of the measured value of CCL17 to the measurement value of CXCL11 (hereinafter also referred to as "CCL17/CXCL11"), the ratio of the measured value of CXCL11 to the measured value of CCL17 (hereinafter also referred to as "CXCL11/CCL17"), the ratio of the measured value of CCL22 to the measured value of CXCL11 (hereinafter also referred to as "CCL22/CXCL11"), the ratio of the measured value of CXCL11 to the measured value of CCL22 (hereinafter also referred to as "CXCL11/CCL22"), the ratio of the measured value of CCL22 to the measured value of CXCL9 (hereinafter, also referred to as "CCL22/CXCL9"), and the ratio of the measured value of CXCL9 to the measured value of CCL22 (hereinafter, also referred to as "CXCL9/CCL22") are preferable. Among them, CCL17/CXCL9, CCL17/CXCL11, and CCL22/CXCL11 are particularly preferable. The ratio may be expressed as a percentage by multiplying the values of these ratios by 100.

In the present embodiment, the value calculated using the measured values of two or more biomarkers may be a value obtained by a known multivariate analysis. The analysis of the measured value of the biomarker by multivariate analysis can be performed, for example, by a cluster analysis, a multiple logistic analysis, and a decision tree.

The means for measuring the biomarker is not particularly limited as long as information reflecting the amount or concentration of the biomarker in the biological sample or the measurement sample prepared from the biological sample can be obtained. In the present embodiment, a method of capturing the biomarker using a substance that can specifically bind to the biomarker is preferable. The biomarker captured by such a substance can be detected by a method known in the art to measure the biomarker contained in the biological sample.

Examples of the substance that can specifically bind to the biomarker include an antibody, an aptamer, and a receptor protein, and the antibody is particularly preferable among them. An antibody to each of the above chemokines is known and generally available. The antibody to the biomarker is not particularly limited as long as it is an antibody that can specifically bind to the biomarker. Such an antibody may be a monoclonal antibody, a polyclonal antibody, and a fragment of them (for example, Fab and F(ab')2). Furthermore, a generally commercially available antibody may be used.

The method for measuring the biomarker using the antibody is not particularly limited and can be appropriately selected from a known immunological measurement method. Examples of such a measurement method include an enzyme-linked immunosorbent assay (ELISA) and Western blotting. Among them, the ELISA is preferable. The kind of the ELISA may be any of a sandwich method, a competitive method, a direct method, and the like, but the sandwich method is particularly preferable. As an example, the case where the biomarker in the biological sample is measured by the sandwich ELISA is described below.

First, a complex containing a biomarker, an antibody for capturing the biomarker (hereinafter, also referred to as a "capture antibody"), and an antibody for detecting the biomarker (hereinafter, also referred to as a "detection antibody") is formed on a solid phase. The complex can be formed by mixing a biological sample that may contain a biomarker, the capture antibody, and the detection antibody. Then, the solution containing the complex can be contacted with a solid phase that can capture the capture antibody to form the above complex on the solid phase. Alternatively, a solid phase in which the capture antibody is immobilized in advance may be used. That is, the above complex can be formed on the solid phase by contacting the solid phase on which the capture antibody is immobilized, the biological sample, and the detection antibody. When both the capture antibody and the detection antibody are monoclonal antibodies, it is preferable that their epitopes are different from each other.

The aspect of immobilizing the capture antibody on the solid phase is not particularly limited. For example, the capture antibody and the solid phase may be directly bound, or the capture antibody and the solid phase may be indirectly bound through another substance. Examples of the direct binding include physical adsorption. Examples of the indirect binding include binding through a combination of biotins including biotin and biotin analogs such as desthiobiotin and avidins including avidin and avidins such as streptavidin and tamavidin (registered trademark). In this case, the capture antibody is modified with biotin in advance, and the avidins are bound to the solid phase in advance, so that the capture antibody and the solid phase can be indirectly bound to each other through the binding between the biotins and the avidins.

The material of the solid phase is not particularly limited, and for example, can be selected from an organic polymer compound, an inorganic compound, and a biopolymer. Examples of the organic polymer compound include latex, a polystyrene, and a polypropylene. Examples of the inorganic compound include magnetic a substance (iron oxide, chromium oxide, ferrite, and the like), silica, alumina, and glass. Examples of the biopolymer include an insoluble agarose, an insoluble dextran, gelatin, and a cellulose. Two or more kind of these may be used in combination. The shape of the solid phase is not particularly limited, and examples of the shape include a particle, a membrane, a microplate, a microtube, and a test tube. Among them, a particle is preferable, and a magnetic particle is particularly preferable.

In the present embodiment, B/F (Bound/Free) separation may be performed between forming the complex and detecting the complex to remove an unreacted free component that do not form the complex. The unreacted free component refers to a component that does not form the complex. For example, a capture antibody and a detection antibody that did not bind to a biomarker can be mentioned. The means for the B/F separation is not particularly limited, when the solid phase is a particle, B/F separation can be performed by recovering only the solid phase in which the complex is captured by centrifugation. When the solid phase is a container such as the microplate or microtube, the B/F separation can be performed by removing the liquid containing the unreacted free component. When the solid phase is the magnetic particle, the B/F separation can be performed by suctioning and removing the liquid containing the unreacted free component with a nozzle while the magnetic particle is magnetically constrained by a magnet. The manner of B/F separation is preferable from the viewpoint of automation. After removing the unreacted free component, the solid phase capturing the complex may be washed with a suitable aqueous medium such as PBS.

Then, the biomarker contained in the biological sample can be measured by detecting the complex formed on the solid phase by a method known in the art. For example, when an antibody labeled with a labeling substance is used as the detection antibody, the biomarker in the biological sample can be measured by detecting the signal generated by the labeling substance. Alternatively, when a labeled secondary antibody to the detection antibody is used, the biomarker in the biological sample can be measured in the same manner.

Furthermore, as an example of the method for measuring the biomarker using an antibody, the immune complex transfer method described in Japanese Laid-Open Patent Publication No. HI-254868 can also be used.

As used herein, "detecting a signal" includes qualitatively detecting the presence or absence of a signal, quantifying the signal intensity, and semi-quantitatively detecting the signal intensity. Semi-quantitative detection means that the signal intensity is indicated in a stepwise way, such as "no signal generated", "weak", "medium", and "strong". In the present embodiment, it is preferable to detect the signal intensity quantitatively or semi-quantitatively.

The labeling substance is not particularly limited as long as a detectable signal is generated. For example, the labeling substance may be a substance that generates a signal by itself (hereinafter, also referred to as a "signal generating substance") or may be a substance that catalyzes a reaction of another substance to generate a signal. Examples of the signal generating substance include a fluorescent substance and a radioactive isotope. Examples of the substance that catalyzes a reaction of another substance to generate a detectable signal include an enzyme. Examples of the enzyme include alkaline phosphatase, peroxidase, β-galactosidase, and luciferase. Examples of the fluorescent substance include fluorescent dyes such as fluorescein isothiocyanate (FITC), rhodamine, and Alexa Fluor (registered trademark), and fluorescent proteins such as GFP. Examples of the radioactive isotope include ¹²⁵I, ¹⁴C, and ³²P. Among them, as the labeling substance, the enzyme is preferable, and alkaline phosphatase and peroxidase are particularly preferable.

The method itself for detecting the signal is known in the art. In the present embodiment, the measurement method according to a kind of the signal derived from the above labeling substance may be appropriately selected. For example, when the labeling substance is an enzyme, the measurement can be carried out by measuring signals such as light and color generated by reacting a substrate with the enzyme using a known device such as a spectrophotometer.

The substrate of the enzyme can be appropriately selected from a known substrate according to a kind of the enzyme. When alkaline phosphatase is used as the enzyme, examples of the substrate include chemiluminescence substrates such as CDP-Star (registered trademark) (disodium 4-chloro-3-(methoxyspiro[1,2-dioxetane-3,2'-(5'-chloro)trixhiro][3.3.1.13,7]decane]-4-yl)phenyl phosphate) and CSPD(registered trademark) (disodium 3-(4-methoxyspiro[1,2-dioxetane-3,2-(5'-chloro)tricyclo[3.3.1.13,7]decane]-4-yl)phenyl phosphate), and coloring substrates such as 5-bromo-4-chloro-3-indrill phosphate (BCIP), disodium 5-bromo-6-chloro-indrill phosphate, and p-nitrophenyl phosphate. When peroxidase is used as the enzyme, examples of the substrate include chemiluminescence substrates such as luminol and its derivative, and coloring substrates such as 2,2'-azinobis(3-ethylbenzothiazolin-6-ammonium sulfonate) (ABTS), 1,2-phenylenediamine (OPD), and 3,3',5,5'-tetramethylbenzidine (TMB).

When the labeling substance is the radioactive isotope, a radiation as a signal can be measured using a known device such as a scintillation counter. When the labeling substance is the fluorescent substance, fluorescence as a signal can be measured using a known device such as a fluorescent microplate reader. The excitation wavelength and the fluorescence wavelength can be appropriately determined according to a kind of the fluorescent substance used.

A detection result of the signal can be used as a measurement result of the biomarker. For example, when quantifying the signal intensity, a measured value itself of the signal intensity or a value obtained from the measured value can be used as the measurement result of the biomarker. Examples of the value obtained from the measured value of the signal intensity include a value obtained by subtracting a measured value of the negative control sample or a background value from the measured value. Furthermore, the measured value of the signal intensity may be applied to the calibration curve to determine a value of the amount or concentration of the biomarker. The negative control sample can be appropriately selected, and examples of the negative control sample include a biological sample obtained from a healthy person.

In the present embodiment, it is preferable to measure a free protein marker contained in the biological sample by a sandwich ELISA using the capture antibody immobilized on the magnetic particle and the detection antibody labeled with the labeling substance. In this case, the measurement may be performed using a generally commercially available fully automatic immunoassay device such as HISCL series (manufactured by SYSMEX CORPORATION).

In the present embodiment, the measurement result of the biomarker is an index of the risk of a decrease in the respiratory function of the patient with interstitial pneumonia. For example, as shown in Examples, a group of patients with a high concentration of CCL17 in the biological sample has a higher risk of a decrease in a respiratory function than a group of patients with a low concentration of CCL17. Furthermore, the group of patients having a low concentration of CXCL9 or CXCL11 in the biological sample has a higher risk of a decrease in a respiratory function than the group of patients having a high concentration of CXCL9 or CXCL11. Therefore, in the present embodiment, the measurement result of the biomarker can be obtained as information on the risk of a decrease in the respiratory function of the patient with interstitial pneumonia. For example, the measurement result of the biomarker may be the measured value of CCL17, the ratio of the measured value of CCL17 to the measured value of CXCL9 (CCL17/CXCL9), or the ratio of the measured value of CCL17 to the measured value of CXCL11 (CCL17/CXCL11). When the measured value or the value of the ratio is higher than the predetermined threshold corresponding to each value, it is suggested that the risk of a decrease in a respiratory function is high. When the measured value of CCL17, the value of CCL17/CXCL9, or the value of CCL17/CXCL11 are lower than the predetermined threshold corresponding to each value, it is suggested that the risk of a decrease in a respiratory function is low.

Furthermore, the measurement result of the biomarker may be the measured value of CXCL9, the measured value of CXCL11, the ratio of the measured value of CXCL9 to the measured value of CCL17 (CXCL9/CCL17), or the ratio of the measured value of CXCL11 to the measured value of CCL17 (CXCL11/CCL17). When the measured value or the value of the ratio is lower than the predetermined threshold corresponding to each value, it is suggested that the risk of a decrease in a respiratory function is high. When the measured value of CXCL9, the measured value of CXCL11, the value of CXCL9/CCL17, or the value of CXCL11/CCL17 is higher than the predetermined threshold corresponding to each value, it is suggested that the risk of a decrease in a respiratory function is low.

When the measured values of CXCL10 and/or CCL22 readings are further obtained, the measurement result of the biomarker may be the value of the ratio of the measured value of CCL17 to the measured value of CXCL10 (CCL17/CXCL10), the ratio of the ratio of the CCL22 to the ratio of CXCL11 (CCL22/CXCL11), or the ratio of the measured value of CCL22 to the measured value of CXCL9 (CCL22/CXCL9). When the value of the ratio is higher than the predetermined threshold corresponding to each value, it is suggested that the risk of a decrease in a respiratory function is high. When the value of the ratio is lower than the predetermined threshold corresponding to each value, it is suggested that the risk of a decrease in a respiratory function is low.

The measurement result of the biomarker may be the value of the ratio of the measured value of CXCL10 to the measured value of CCL17 (CXCL10/CCL17), the ratio of the measured value of CXCL11 to the measured value of CCL22 (CXCL11/CCL22), or the ratio of the measured values of CXCL9 to the CCL22 measured value (CXCL9/CCL22). When the value of the ratio is lower than the predetermined threshold corresponding to each value, it is suggested that the risk of a decrease in a respiratory function is high. When the value of the ratio is higher than the predetermined threshold corresponding to each value, it is suggested that the risk of a decrease in a respiratory function is low.

In the present embodiment, the risk of a decrease in a respiratory function may be a risk of a decrease in a test value indicating a respiratory function after a lapse of a predetermined period from collection of the biological sample. For example, the risk of a decrease in a respiratory function may be the risk of a decrease in vital capacity, for example, 6 months after the collection of the biological sample. The risk of a decrease in vital capacity may be a risk that the vital capacity, for example, 6 months after the collection of the biological sample, decreases, for example, by 5% or more from the vital capacity at the time of collection of the biological sample.

The measurement result of the biomarker can be used to determine the risk of a decrease in a respiratory function. A medical staff such as a doctor may use the measurement result to determine the risk of a decrease in a respiratory function or may combine the measurement result with other information to determine the risk of a decrease in a respiratory function. Here, the "other information" includes vital capacity, lung diffusivity, the findings of a X-ray image or a CT image, and other medical findings.

### [2. Method for determining risk of decrease in respiratory function]

In the present invention, the measurement result of the biomarker obtained by the above method can be used to determine the risk of a decrease in a respiratory function of a patient with interstitial pneumonia. In the method for determining the risk of a decrease in a respiratory function of a patient with interstitial pneumonia in the present invention (hereinafter, also referred to as "determination method"), first, at least one biomarker in a biological sample of a patient with interstitial pneumonia is measured. The details of the measurement are the same as those described for the method for obtaining information on the risk of a decrease in a respiratory function in the present invention.

In the determination method of the present invention, the risk of a decrease in a respiratory function of a patient with interstitial pneumonia is determined based on the measurement result of the biomarker. The details of the risk of a decrease in a respiratory function are the same as those described for the method for obtaining information on the risk of a decrease in the respiratory function of the present invention. In a preferred embodiment, the risk of a decrease in a respiratory function of a patient is determined based on a measured value of one biomarker or a value of a ratio of the measured values of two biomarkers, and the result of comparing a predetermined threshold corresponding to each value.

For example, when the measurement result of the biomarker is the measured value of CCL17, the ratio of the measured value of CCL17 to the measured value of CXCL9 (CCL17/CXCL9), the ratio of the measured value of CCL17 to the measured value of CXCL11 (CCL17/CXCL11), the ratio of the measured value of CXCL11 to the measured value of CCL22 (CCL22/CXCL11), or the ratio of the measured value of CCL22 to the measured value of CXCL9 (CCL22/CXCL9), and the measured value of CCL17, the value of CCL17/CXCL9, the value of CCL17/CXCL11, the value of CCL22/CXCL11, or the value of CCL22/CXCL9 is higher than the predetermined threshold corresponding to each value, it is determined that the risk of a decrease in a respiratory function is high. Furthermore, when the measured value of CCL17, the value of CCL17/CXCL9, the value of CCL17/CXCL11, the value of CCL22/CXCL11, or the value of CCL22/CXCL9 is lower than the predetermined threshold corresponding to each value, it is determined that the risk of a decrease in a respiratory function is low. In the present embodiment, when the above measured value or ratio value is equal to the predetermined threshold corresponding to each value, it may be determined that the risk of a decrease in a respiratory function is low or the risk of a decrease in a respiratory function is high. That is, when the measured value of CCL17, the value of CCL17/CXCL9, the value of CCL17/CXCL11, the value of CCL22/CXCL11, or the value of CCL22/CXCL9 is equal to or higher than the predetermined threshold corresponding to each value, it may be determined that the risk of a decrease in a respiratory function is high. Alternatively, when the measured value of CCL17, the value of CCL17/CXCL9, the value of CCL17/CXCL11, the value of CCL22/CXCL11, or the value of CCL22/CXCL9 is equal to or lower than the predetermined threshold corresponding to each value, it may be determined that the risk of a decrease in a respiratory function is low.

For example, when the measurement result of the biomarker is the measured value of CXCL9, the measured value of CXCL11, the ratio of the measured value of CCL17 to the measured value of CXCL9 (CXCL9/CCL17), the ratio of the measured value of CCL17 to the measured value of CXCL11(CXCL11/CCL17), the ratio of the measured value of CXCL11 to the measured value of CCL22 (CXCL11/CCL22), or the ratio of the measured value of CXCL9 to the measured value of CCL22 (CXCL9/CCL22), and the measured value of CXCL9, the measured value of CXCL11, the value of CXCL9/CCL17, the value of CXCL11/CCL17, the value of CXCL11/CCL22, or the valued of CXCL9/CCL22 is lower than the predetermined threshold corresponding to each value, it is determined that the risk of a decrease in a respiratory function is high. When the measured value of CXCL9, the measured value of CXCL11, the value of CXCL9/CCL17, the value of CXCL11/CCL17, the value of CXCL11/CCL22, or the value of CXCL9/CCL22 is higher than the predetermined threshold corresponding to each value, it is determined that the risk of a decrease in a respiratory function is low. In the present embodiment, when the above measured value or ratio value is equal to the predetermined threshold corresponding to each value, it may be determined that the risk of a decrease in a respiratory function is low or the risk of a decrease in a respiratory function is high. That is, when the measured value of CXCL9, the measured value of CXCL11, the value of CXCL9/CCL17, the value of CXCL11/CCL17, the value of CXCL11/CCL22, or the value of CXCL9/CCL22 is or equal to or lower than the predetermined threshold corresponding to each value, it may be determined that the risk of a decrease in a respiratory function is high. Alternatively, when the measured value of CXCL9, the measured value of CXCL11, the value of CXCL9/CCL17, the value of CXCL11/CCL17, the value of CXCL11/CCL22, or the value of CXCL9/CCL22 is equal to or higher than the predetermined threshold corresponding to each value, it may be determined that the risk of a decrease in a respiratory function is low.

A patient who is determined to have a high risk of a decrease in a respiratory function may be determined to have a high possibility of a decrease by 5% or more in a respiratory function (for example, vital capacity) 6 months after the collection of the biological sample. On the other hand, the patient who is determined to have a low risk of a decrease in a respiratory function may be determined to have a low possibility of a decrease by 5% or more in a respiratory function (for example, vital capacity) 6 months after the collection of the biological sample.

As described above, the determination method of the present invention can provide a medical staff such as a doctor with information that assists in determining the risk of a decrease in a respiratory function. Appropriate treatment can be performed on a patient with interstitial pneumonia determined to have a high risk of a decrease in a respiratory function by the determination method of the present invention to prevent the progression of the disease and prevent decrease in the respiratory function. For example, in the treatment of hypersensitivity pneumonia, antigen avoidance and antiinflammatory therapy are mainly performed, and when it is determined that the risk of a decrease in a respiratory function is high by the determination method of the present invention, further administration of an antifibrotic drug may be considered.

The predetermined threshold is not particularly limited and can be set as appropriate. For example, a predetermined threshold may be set empirically by accumulating data of a measured value of the biomarker of a patient with interstitial pneumonia. Alternatively, a predetermined threshold may be set as follows. First, biological samples are collected from a plurality of patients with interstitial pneumonia, the biomarkers are measured to obtain the measured values of the biomarkers. After a predetermined period of time (for example, 6 months) passed since the collection of the biological samples, it is confirmed whether or not the respiratory function of these patients was decreased. The above data is classified into data of a group of patients with decreased respiratory function and data of a group of patients without decreased respiratory function. Then, a value that can most accurately distinguish between the group of patients with decreased respiratory function and the group of patients without decreased respiratory function is obtained, and the value is set as the predetermined threshold. In setting the threshold value, it is preferable to consider sensitivity, specificity, positive predictive value, negative predictive value, and the like.

### [3. Reagent kit]

The scope of the present invention also includes a reagent kit used in the method and determination method of the present invention. That is, provided is a reagent kit containing a reagent containing a substance that can specifically bind to CCL17, and/or a reagent containing a substance that can specifically bind to CXCL9, and/or a reagent containing a substance that can specifically bind to CXCL11 (hereinafter, also referred to as "reagent kit"). The details of the substances that can specifically bind to each of CCL17, CXCL9, and CXCL11 are the same as those described for the substances that can specifically bind to the biomarker used in the method of the present invention.

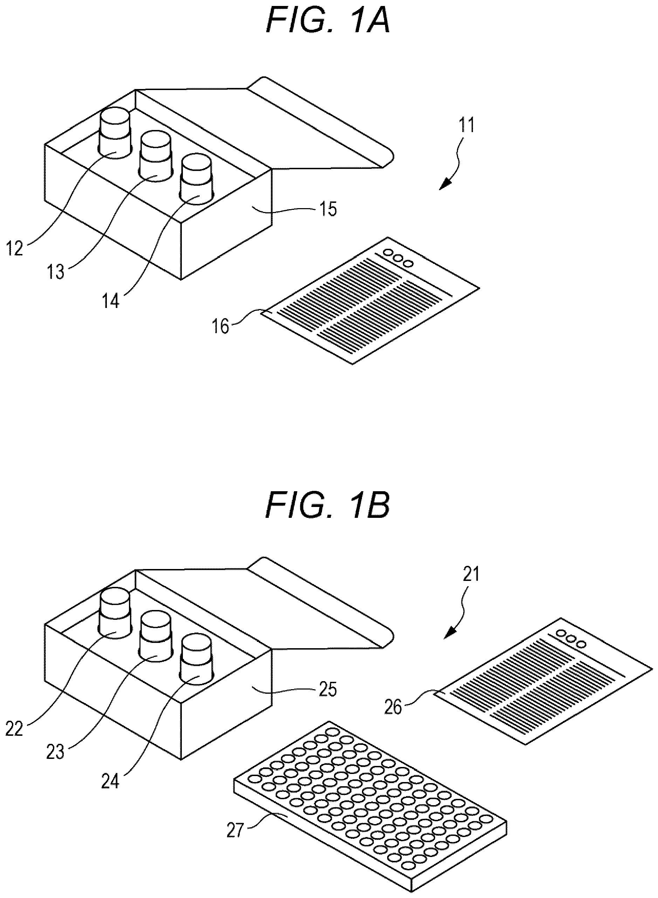

In the present invention, the container containing each reagent may be packed in a box and provided to the user. A package leaflet may be included in the box. The package leaflet may describe the configuration of the reagent kit, usage, the relativity between the measurement result obtained by the reagent kit and the risk of a decrease in a respiratory function, and the like. An example of such a reagent kit is shown in the drawings. With reference to Fig. 1A, 11 shows a reagent kit, 12 shows a first container containing a reagent containing a substance that can specifically bind to CCL17, and 13 shows a second container containing a reagent containing a substance that can specifically bind to CXCL9, 14 shows a third container containing a reagent containing a substance that can specifically bind to CXCL11, 15 is a packing box, and 16 is a package leaflet.

The reagent kit of the present invention may further include a solid phase for immobilizing a substance that can specifically bind to the biomarker. With reference to Fig. 1B, 21 shows a reagent kit, 22 shows a first container containing a reagent containing a substance that can specifically bind to CCL17, 23 shows a second container that can specifically bind to CXCL9, 24 shows a third container containing a reagent containing a substance that can specifically bind to CXCL11, 25 shows a packing box, 26 shows a package leaflet, and 27 shows a solid phase. In the figure, the solid phase is represented as a 96-well microplate, but the invention is not limited to the example.

The reagent kit of the present invention may further include at least one reagent selected from a group consisting of a reagent containing a substance that can specifically bind to CCL22 and a reagent that contains a substance that can specifically bind to CXCL10.

In the present embodiment, the reagent containing a substance that can specifically bind to CCL17 is preferably a combination of a reagent containing a capture antibody to CCL17 and a reagent containing a detection antibody to CCL17. Furthermore, the reagent containing a substance that can specifically bind to CXCL9 is preferably a combination of a reagent containing a capture antibody to CXCL9 and a reagent containing a detection antibody to CXCL9. Furthermore, the reagent containing a substance that can specifically bind to CXCL11 is preferably a combination of a reagent containing a capture antibody to CXCL11 and a reagent containing a detection antibody to CXCL11. The detection antibody may be labeled with a labeling substance. when the labeling substance is an enzyme, the reagent kit may include a substrate for the enzyme. The details of the capture antibody, the detection antibody, the labeling substance, and the substrate are the same as those described for the method of the present invention described above. The form of the capture antibody, the detection antibody, the labeling substance, and the substrate is not particularly limited, and may be a solid (for example, powder, a crystal, and a lyophilized product) or a liquid (for example, solution, suspension, and emulsion).

In the present embodiment, the reagent kit may include a calibrator for each biomarker. Examples of the calibrator include a calibrator for CCL17 determination, a calibrator for CXCL9 determination, and a calibrator for CXCL11 determination. The calibrator for CCL17 determination may include, for example, a buffer containing no CCL17 (negative control) and a buffer containing CCL17 at a known concentration. The calibrator for CXCL9 determination may include, for example, a buffer containing no CXCL9 (negative control) and a buffer containing CXCL9 at a known concentration. The calibrator for CXCL11 determination may include, for example, a buffer containing no CXCL11 (negative control) and a buffer containing CXCL11 at a known concentration. Examples of another calibrator include a calibrator including a buffer containing none of CCL17, CXCL9, and CXCL11 (negative control), a buffer containing CCL17 at a known concentration, a buffer containing CXCL9 at a known concentration, and a buffer containing CXCL11 at a known concentration. Examples of another calibrator include a calibrator including a buffer containing none of CCL17, CXCL9, and CXCL11 (negative control) and a buffer containing CCL17, CXCL9, and CXCL11 at known concentrations, respectively.

### [4. Device and computer program]

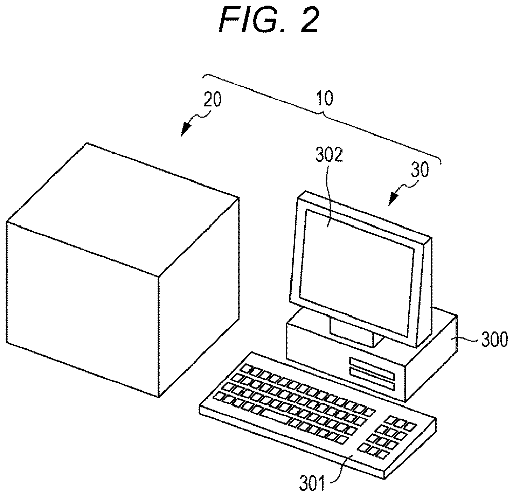

The scope of the present invention also includes a device and a computer program for carrying out the method for determining a risk of a decrease in a respiratory function according to the present invention. An example of the device for determining a risk of a decrease in a respiratory function according to the present invention is described with reference to the drawings. However, the present invention is not limited to the embodiment shown in the example. The determination device 10 shown in Fig. 2 includes a measuring device 20 and a computer system 30 connected to the measuring device 20.

The kind of the measuring device is not particularly limited and can be appropriately selected according to the method for measuring the biomarker. In the example shown in Fig. 2, the measuring device 20 is a generally commercially available automatic immunoassay device capable of detecting a chemiluminescent signal generated by a sandwich ELISA using a magnetic particle on which a capture antibody is immobilized and an enzyme-labeled detection antibody. However, the present invention is not limited to the example. When the biomarker is measured by the ELISA, the measuring device is not particularly limited as long as it can detect a signal based on a labeling substance used.

When a reagent containing a magnetic particle on which a capture antibody is immobilized, a reagent containing an enzyme-labeled detection antibody, and a biological sample collected from a subject are set in the measuring device 20, the measuring device 20 executes an antigen-antibody reaction using each reagent, obtains a chemiluminescent signal as optical information based on an enzyme-labeled antibody specifically binding to a biomarker, and sends obtained optical information to computer system 30.

The computer system 30 includes a computer main body 300, an input part 301, and a display 302. The display 302 displays specimen information, determination results, and the like. The computer system 30 receives optical information from the measuring device 20. Then, the processor of the computer system 30 executes a computer program installed on a hard disk 313 for determining the risk of a decrease in a respiratory function based on the optical information. As shown in Fig. 2, the computer system 30 may be a device separate from the measuring device 20 or a device including the measuring device 20. In the latter case, the computer system 30 itself may be the determination device 10. A generally commercially available automatic immunoassay device may be installed with a computer program for determining the risk of a decrease in a respiratory function.

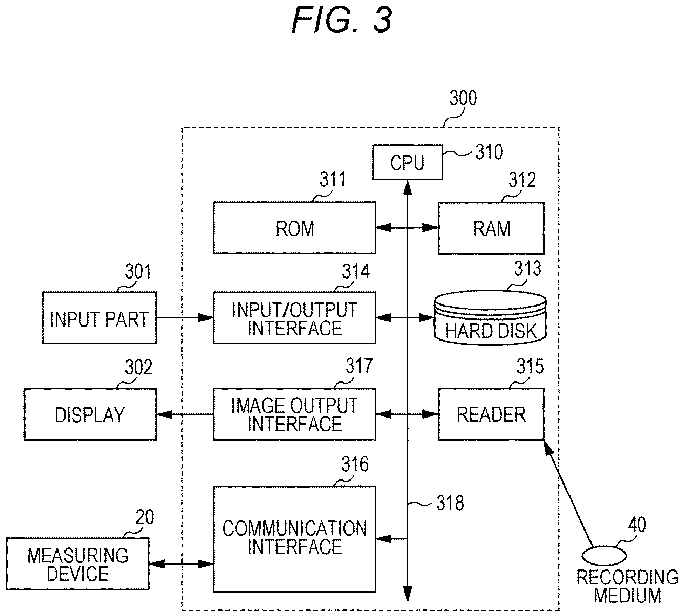

With reference to Fig. 3, the computer main body 300 includes a CPU (Central Processing Unit) 310, a ROM (Read Only Memory) 311, a RAM (Random Access Memory) 312, a hard disk 313, an input/output interface 314, a reader 315, a communication interface 316, and an image output interface 317. The CPU 310, ROM 311, RAM 312, hard disk 313, input/output interface 314, reader 315, communication interface 316, and image output interface 317 are connected by a bus 318 so that data communication is possible. Further, the measuring device 20 is connected to the computer system 30 by the communication interface 316 so that communication is possible.

The CPU 310 can execute a program stored in the ROM 311 or the hard disk 313 and a program loaded in the RAM 312. The CPU 310 obtains the measured value of the biomarker based on the optical information obtained from the measuring device 20. When two or more biomarkers are measured, the CPU 310 may obtain a value calculated using the measured values of the two or more biomarkers. The details of these values are the same as those described for the method of the present invention. The CPU 310 determines the risk of a decrease in a respiratory function based on the obtained value and a predetermined threshold stored in the ROM 311 or the hard disk 313. The CPU 310 outputs the determination result and displays it on the display 302.

The ROM 311 has a mask ROM, a PROM, an EPROM, an EEPROM, and the like. The ROM 311 records the computer program executed by the CPU 310 and data used for executing the computer program.

The RAM 312 has an SRAM, a DRAM, and the like. The RAM 312 is used to read the programs recorded in the ROM 311 and the hard disk 313. The RAM 312 is also used as a work area for CPU 310 when executing these programs.

The hard disk 313 is installed with an operating system for the CPU 310 to execute, a computer program such as an application program (a program for determining the risk of a decrease in a respiratory function), and data used for executing the computer program.

The reader 315 has a flexible disk drive, a CD-ROM drive, a DVD-ROM drive, and the like. The reader 315 can read a program or data recorded on a portable recording medium 40.

The input/output interface 314 has, for example, a serial interface such as USB, IEEE1394, and RS-232C, a parallel interface such as SCSI, IDE, and IEEE1284, and an analog interface having a D/A converter, an A/D converter, and the like. The input part 301 such as a keyboard and a mouse is connected to the input/output interface 314. The operator can input various commands to the computer main body 300 by the input part 301.

The communication interface 316 is, for example, an Ethernet (registered trademark) interface. The computer main body 300 can also transmit print data to a printer or the like with the communication interface 316.

The image output interface 317 is connected to the display 302 having an LCD, a CRT, and the like. As a result, the display 302 can output a video signal corresponding to an image data given from the CPU 310. The display 302 displays an image (screen) according to the input video signal.

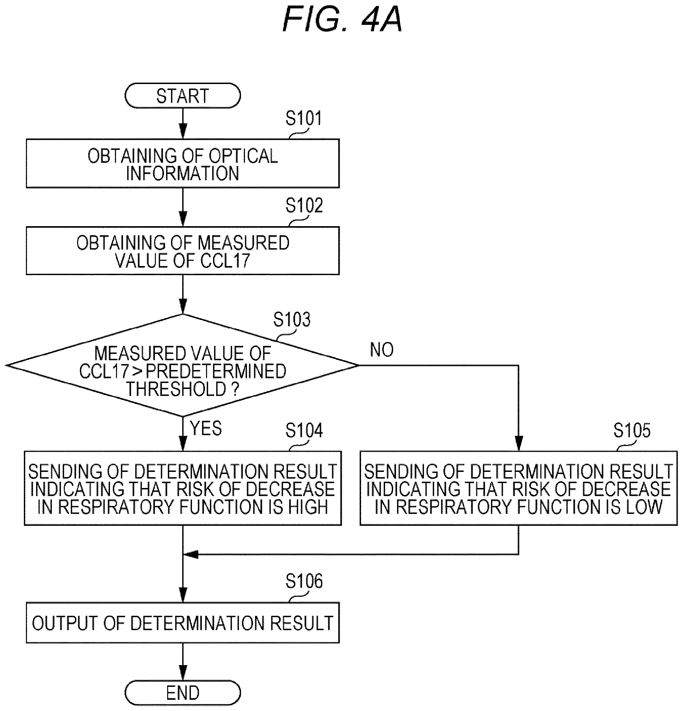

With reference to Fig. 4A, a processing procedure for determining the risk of a decrease in a respiratory function, which is executed by the determination device 10, is described. Specifically, an example is described in which the measured value of CCL17 is obtained from the intensity of the chemiluminescent signal generated by the sandwich ELISA, and the determination is made using the obtained measured value and a predetermined threshold corresponding to it. However, the present invention is not limited to the example.

In step S101, the CPU 310 obtains optical information (chemiluminescent signal) from the measuring device 20 and stores it in the hard disk 313. In step S102, the CPU 310 obtains the measured value of CCL17 from the obtained optical information and stores it in the hard disk 313. In step S103, the CPU 310 compares the obtained measured value with a predetermined threshold stored in the hard disk 313. When the measured value is higher than the predetermined threshold, the processing proceeds to step S104, and the determination result indicating that the patient has a high risk of a decrease in a respiratory function is stored in the hard disk 313. When the measured value is equal to or lower than the predetermined threshold, the processing proceeds to step S105, and the determination result indicating that the patient has a low risk of a decrease in a respiratory function is stored in the hard disk 313. In step S106, the CPU 310 outputs the determination result, displays it on the display 302, or prints it with a printer. As a result, it is possible to provide information to a doctor or the like to assist in determining the risk of a decrease in a respiratory function.

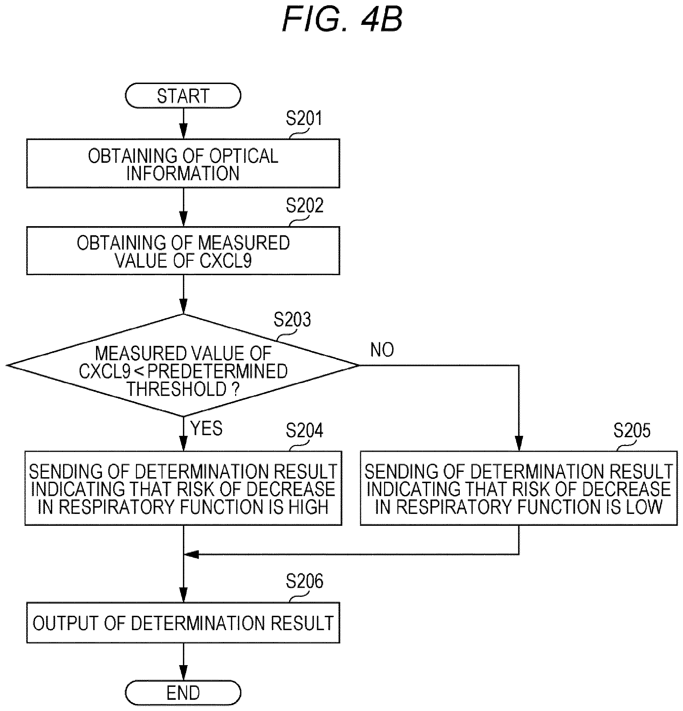

With reference to Fig. 4B, a case where the determination is made based on the measured value of CXCL9 is described. Steps S201 and S206 are similar to those described for steps S101 and S106 above, respectively. In step S202, the CPU 310 obtains the measured value of CXCL9 from the obtained optical information and stores it in the hard disk 313. In step S203, the CPU 310 compares the obtained measured value with a predetermined threshold stored in the hard disk 313. When the measured value is lower than the predetermined threshold, the processing proceeds to step S204, and the determination result indicating that the patient has a high risk of a decrease in a respiratory function is stored in the hard disk 313. When the measured value is equal to or higher than the predetermined threshold, the processing proceeds to step S205, and the determination result indicating that the patient has a low risk of a decrease in a respiratory function is stored in the hard disk 313.

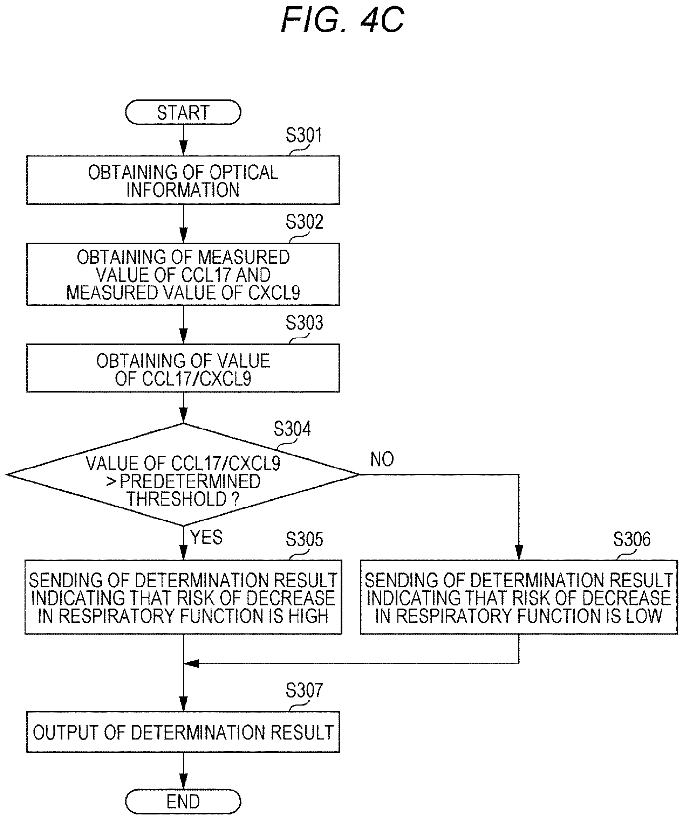

With reference to Fig. 4C, a case where CCL17 and CXCL9 are measured and a determination is made based on a value calculated using the measured values of these biomarkers is described. Steps S301 and S307 are similar to those described for steps S101 and S106 above, respectively. In step S302, the CPU 310 obtains the measured value of CCL17 and the measured value of CXCL9 from the obtained optical information and stores them in the hard disk 313. In step S303, the CPU 310 obtains the value of CCL17/CXCL9 by dividing the measured value of CCL17 by the measured value of CXCL9. In step S304, the CPU 310 compares the obtained value of CCL17/CXCL9 with a predetermined threshold stored in the hard disk 313. When the value of CCL17/CXCL9 is higher than the predetermined threshold, the processing proceeds to step S305, and the determination result indicating that the patient has a high risk of a decrease in a respiratory function is stored in the hard disk 313. When the value of CCL17/CXCL9 is equal to or lower than the predetermined threshold, the processing proceeds to step S306, and the determination result indicating that the patient has a low risk of a decrease in a respiratory function is stored in the hard disk 313.

In step S303, instead of obtaining the value of CCL17/CXCL9, the value of CXCL9/CCL17 may be obtained by dividing the measured value of CXCL9 by the measured value of CCL17. In this case, the CPU 310 compares the calculated value of CXCL9/CCL17 with the predetermined threshold stored in the hard disk 313. When the value of CXCL9/CCL17 is lower than the predetermined threshold, the CPU 310 stores a determination result indicating that the patient has a high risk of a decrease in a respiratory function in the hard disk 313. When the value of CCL17/CXCL9 is equal to or higher than the predetermined threshold, the CPU 310 stores a determination result indicating that the patient has a low risk of a decrease in a respiratory function in the hard disk 313.

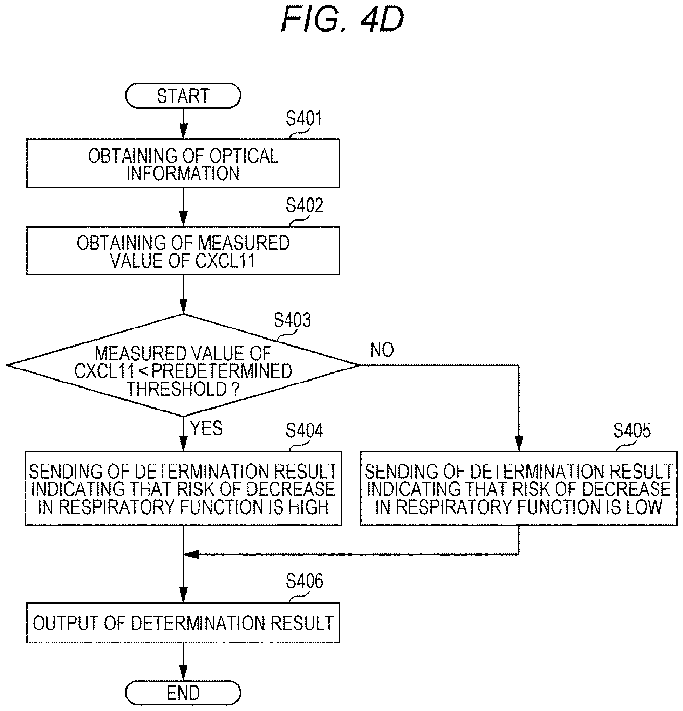

With reference to Fig. 4D, a case where the determination is made based on the measured value of CXCL11 is described. Steps S401 and S406 are similar to those described for steps S101 and S106 above, respectively. In step S402, the CPU 310 obtains the measured value of CXCL11 from the obtained optical information and stores it in the hard disk 313. In step S403, the CPU 310 compares the obtained measured value with a predetermined threshold stored in the hard disk 313. When the measured value is lower than the predetermined threshold, the processing proceeds to step S404, and the determination result indicating that the patient has a high risk of a decrease in a respiratory function is stored in the hard disk 313. When the measured value is equal to or higher than the predetermined threshold, the processing proceeds to step S405, and the determination result indicating that the patient has a low risk of a decrease in a respiratory function is stored in the hard disk 313.

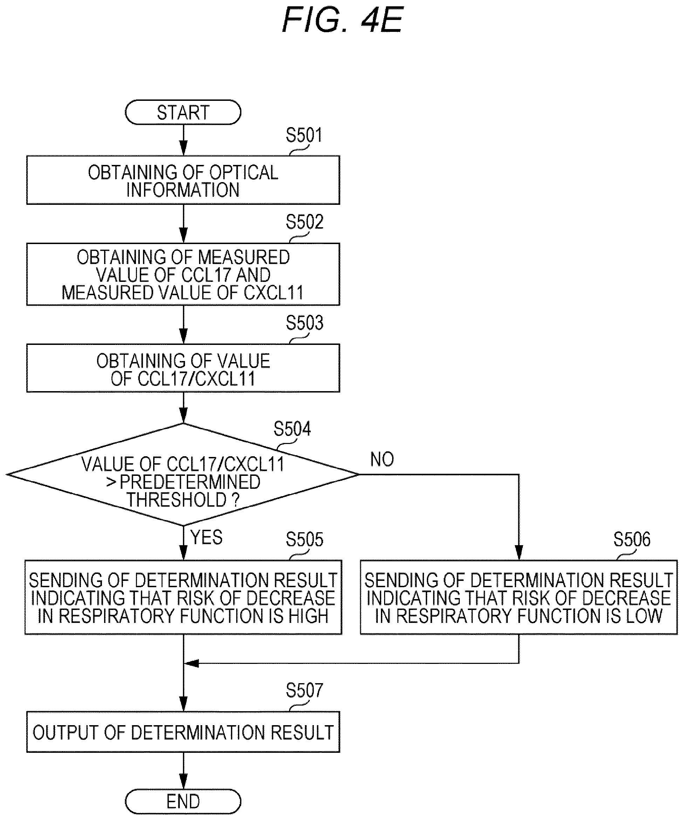

With reference to Fig. 4E, a case where CCL17 and CXCL11 are measured and the determination is made based on a value calculated using the measured values of these biomarkers is described. Steps S501 and S507 are similar to those described for steps S101 and S106 above, respectively. In step S502, the CPU 310 obtains the measured value of CCL17 and the measured value of CXCL11 from the obtained optical information and stores them in the hard disk 313. In step S503, the CPU 310 obtains the value of CCL17/CXCL11 by dividing the measured value of CCL17 by the measured value of CXCL11 In step S504, the CPU 310 compares the obtained value of CCL17/CXCL11 with a predetermined threshold stored in the hard disk 313. When the value of CCL17/CXCL11 is higher than the predetermined threshold, the processing proceeds to step S505, and the determination result indicating that the patient has a high risk of a decrease in a respiratory function is stored in the hard disk 313. When the value of CCL17/CXCL11 is equal to or lower than the predetermined threshold, the processing proceeds to step S506, and the determination result indicating that the patient has a low risk of a decrease in a respiratory function is stored in the hard disk 313.

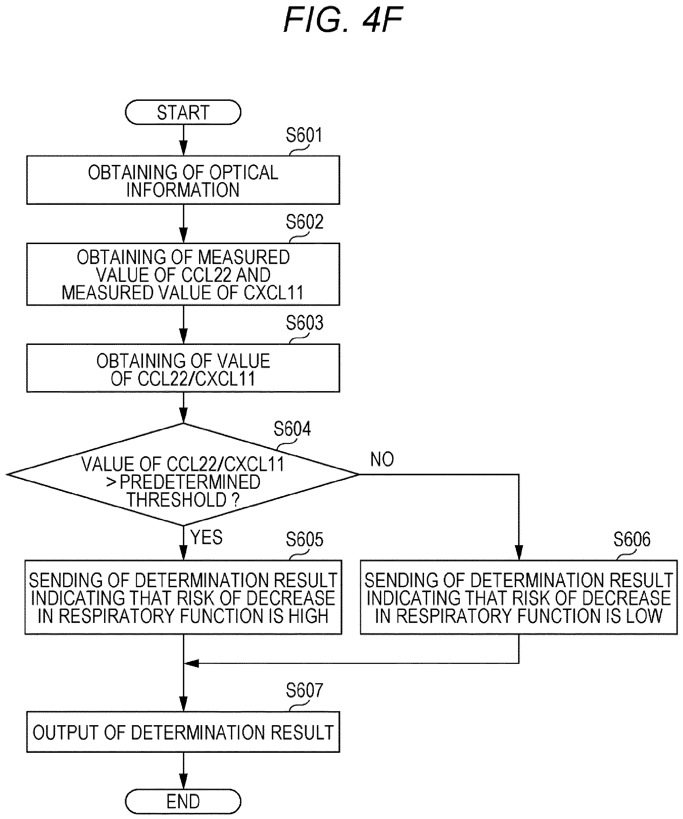

With reference to Fig. 4F, a case where CCL22 and CXCL11 are measured and the determination is made based on a value calculated using the measured values of these biomarkers is described. Steps S601 and S607 are similar to those described for steps S101 and S106 above, respectively. In step S602, the CPU 310 obtains the measured value of CCL22 and the measured value of CXCL11 from the obtained optical information and stores them in the hard disk 313. In step S603, the CPU 310 obtains the value of CCL22/CXCL11 by dividing the measured value of CCL22 by the measured value of CXCL11. In step S604, the CPU 310 compares the obtained value of CCL22/CXCL11 with a predetermined threshold stored in the hard disk 313. When the value of CCL22/CXCL11 is higher than the predetermined threshold, the processing proceeds to step S605, and the determination result indicating that the patient has a high risk of a decrease in a respiratory function is stored in the hard disk 313. When the value of CCL22/CXCL11 is equal to or lower than the predetermined threshold, the processing proceeds to step S606, and the determination result indicating that the patient has a low risk of a decrease in a respiratory function is stored in the hard disk 313.

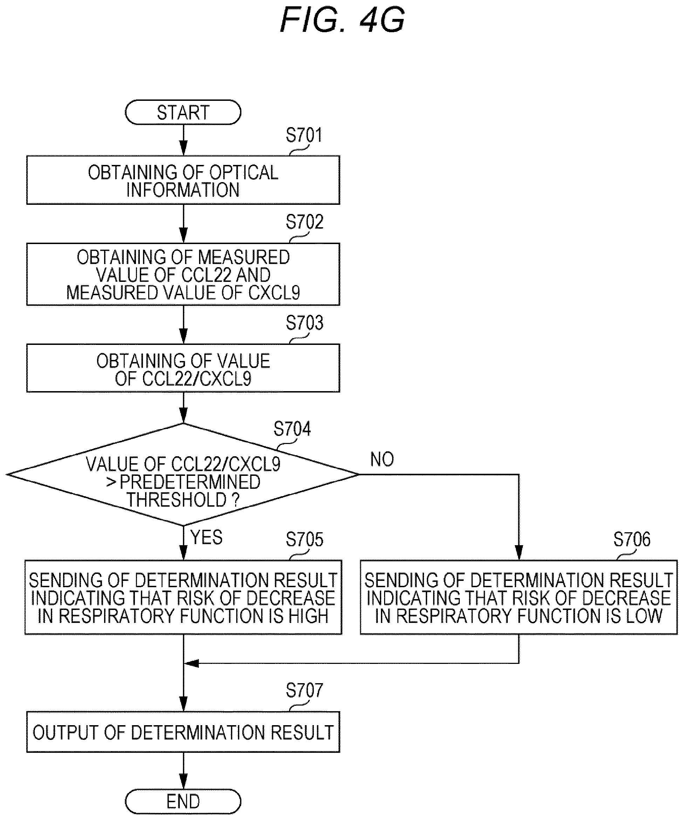

With reference to Fig. 4G a case where CCL22 and CXCL9 are measured and the determination is made based on a value calculated using the measured values of these biomarkers is described. Steps S701 and S707 are similar to those described for steps S101 and S106 above, respectively. In step S702, the CPU 310 obtains the measured value of CCL22 and the measured value of CXCL9 from the obtained optical information and stores them in the hard disk 313. In step S703, the CPU 310 obtains the value of CCL22/CXCL9 by dividing the measured value of CCL22 by the measured value of CXCL9. In step S704, the CPU 310 compares the obtained value of CCL22/CXCL9 with a predetermined threshold stored in the hard disk 313. When the value of CCL22/CXCL9 is higher than the predetermined threshold, the processing proceeds to step S705, and the determination result indicating that the patient has a high risk of a decrease in a respiratory function is stored in the hard disk 313. When the value of CCL22/CXCL9 is equal to or lower than the predetermined threshold, the processing proceeds to step S706, and the determination result indicating that the patient has a low risk of a decrease in a respiratory function is stored in the hard disk 313.

Hereinafter, the present invention is described in detail with reference to Examples, but the present invention is not limited to these Examples. Hereinafter, "HISCL" is a registered trademark of SYSMEX CORPORATION.

### EXAMPLES

### Example 1

### (1) Biological sample

The sera of 61 untreated patients with interstitial pneumonia were used as biological samples. Forty-eight of the 61 patients were tested to confirm the respiratory function (vital capacity) 6 months after serum collection. Patients with a decrease in vital capacity of 5% or more were classified into the "decreased group", and patients with a decrease in vital capacity of less than 5% were classified into the "stable group". Table 1 shows patient information for each group. In the table, pack-year is an index of smoking amount, and is calculated by multiplying the number of cigarette boxes smoked per day by the number of years of smoking. In the table, the age and pack-year for each group are shown by median, first and third quartiles. For the measurement of CCL17 and CXCL9, biological samples obtained from the patients shown in Table 1 were used. For the measurement of CCL22, CXCL10, and CXCL11, biological samples obtained from the patients shown in Table 2 included in the patients shown in Table 1 were used.

**[Table 1]**

| CCL17 and CXCL9 | | | |
|---|---|---|---|
| | Decreased group | Stable group | P |
| Number of persons | 14 | 34 | |
| Age | 60.5 (51.8 - 69.0) | 64.5 (57.3 - 72.0) | 0.216 |
| Sex (Man/Woman) | 8/6 | 14/20 | 0.313 |
| Pack-year | 10.0 (0.0 - 28.0) | 0.0 (0.0 - 25.3) | 0.387 |

**[Table 2]**

| CCL22, CXCL10, and CXCL11 | | | |
|---|---|---|---|
| | Decreased group | Stable group | P |
| Number of persons | 10 | 25 | |
| Age | 60.5 (45.0-69.0) | 69.0 (57.3-74.3) | 0.133 |
| Sex (Man/Woman) | 6/4 | 8/17 | 0.127 |
| Pack-year | 6 (0-24) | 0 (0-10.8) | 0.479 |

### (2) Measurement of biomarkers

### (2.1) Measurement of CCL17

The CCL17 concentration in the serum of each patient was measured with a fully automatic immunoassay device HISCL-5000 (SYSMEX CORPORATION) using a HISCL TARC reagent (SHIONOGI & CO., LTD.). The HISCL TARC reagent includes an R1 reagent containing biotin-labeled mouse anti-human TARC monoclonal antibody, an R2 reagent containing a magnetic particle with streptavidin immobilized on the surface (hereinafter, also referred to as an "STA-bound magnetic particle"), an R3 reagent containing an alkaline phosphatase (ALP)-labeled mouse anti-human TARC monoclonal antibody, a HISCL R4 reagent as a buffer for measurement, a HISCL R5 reagent containing CDP-Star (registered trademark) (Applied Biosystems) which is a chemiluminescent substrate for ALP, a HISCL cleaning solution, and a TARC calibrator were included.

The measurement procedure using HISCL-5000 was as follows. After mixing serum (20 µL) and the R1 reagent (50 µL), the R2 reagent (30 µL) was added. The magnetic particle in the obtained mixed solution was collected to remove the supernatant, and the HISCL cleaning solution (300 µL) was added to wash the magnetic particle. The supernatant was removed, and the R3 reagent (100 µL) was added to the magnetic particle and mixed. The magnetic particle in the obtained mixed solution was collected to remove the supernatant, and the HISCL cleaning solution (300 µL) was added to wash the magnetic particle. The supernatant was removed, the R4 reagent (50 µL) and the R5 reagent (100 µL) were added to the magnetic particle, and the chemiluminescence intensity was measured. The obtained chemiluminescence intensity was applied to the calibration curve to determine the concentration of CCL17.

### (2.2) Measurement of CXCL9

The CXCL9 concentration in the serum of each patient was measured by HISCL-5000 (SYSMEX CORPORATION) using the following R1 to R5 reagents. The measurement procedure for CXCL9 was similar to that for CCL17. Each reagent was prepared as follows.

### - R1 reagent

An anti-MIG monoclonal antibody (Randox Laboratories) was digested with pepsin or IdeS protease by a conventional method to obtain a Fab fragment. The Fab fragment was labeled with biotin by a conventional method and dissolved in 1% bovine serum albumin (BSA) and 0.5% casein-containing buffer to obtain the R1 reagent.

### - R2 reagent

STA-bound magnetic particle (average particle diameter 2 µm) The STA-bound magnetic particle was washed 3 times with 10 mMHEPES buffer (pH 7.5) using streptavidin (2.9 to 3.5 mg) per 1 g of the magnetic particle. The washed STA-bound magnetic particle was added to 10 mM HEPES (pH 7.5) so that the streptavidin concentration was 18 to 22 µg/ml (STA-bound a magnetic particle concentration was 0.48 to 0.52 mg/ml),and the R2 reagent was obtained.

### - R3 reagent

An anti-MIG monoclonal antibody (Randox Laboratories) was digested with pepsin or IdeS protease by a conventional method to obtain a Fab fragment. The Fab fragment was labeled with ALP by a conventional method and dissolved in a buffer containing 1% BSA and 0.5% casein to obtain the R3 reagent.

### - R4 reagent and R5 reagent

As the R4 reagent, HISCL R4 reagent (SYSMEX CORPORATION) was used. As the R5 reagent, HISCL R5 reagent (SYSMEX CORPORATION) was used.

### (2.3) Measurement of CCL22

The CCL22 concentration in serum of each patient was measured by the ELISA. The measurement procedure for CCL22 was as follows. A 2 µg/mL anti-CCL22 mouse monoclonal antibody solution was added to each well of a 96-well plate and the antibody was sensitized to the well. A serum (100 µL) diluted 20-fold with a specimen diluent was added to each well and reacted for 2 hours. After washing each well 6 times with HISCL cleaning solution (300 µL), 100 µL of biotinylated chicken anti-human CCL22 antibody (0.0125 µg/mL, 1% BSA, 0.5% casein, PBS, and pH 7.4) was added and reacted for 2 hours. Each well was washed 6 times with the HISCL cleaning solution (300 µL), and then 100 µL of streptavidin ALP was added and reacted for 20 minutes. After washing each well 6 times with the HISCL cleaning solution (300 µL), 100 µL of a mixture of R4 and R5 reagents at a ratio of 1 : 2 was added to each well, and the luminescence signal was counted with a plate reader. As the anti-CCL22 antibody, Duoset DY336 manufactured by R&D Systems, Inc. was used.

### (2.4) Measurement of CXCL10

The CXCL10 concentration in the serum of each patient was measured by the ELISA. The measurement procedure for CXCL10 was as follows. A 2 µg/mL anti-CXCL10 mouse monoclonal antibody solution was added to each well of a 96-well plate and the antibody was sensitized to the well. A serum (50 µL) diluted 5-fold with a specimen diluent was added to each well and reacted for 2 hours. After washing each well 6 times with the HISCL cleaning solution (300 µL), 50 µL of biotinylated goat anti-human CXCL10 antibody (0.0125 µg/mL, 1% BSA, 0.5% casein, PBS, and pH 7.4) was added and reacted for 2 hours. After washing each well 6 times with the HISCL cleaning solution (300 µL), 50 µL of streptavidin ALP was added and reacted for 20 minutes. After washing each well 6 times with the HISCL cleaning solution (300 µL), 100 µL of a mixture of R4 and R5 reagents at a ratio of 1 : 2 was added to each well, and the luminescence signal was counted with a plate reader. As the anti-CXCL10 antibody, Duoset DY266 manufactured by R&D Systems, Inc. was used.

### (2.5) Measurement of CXCL11

The CXCL11 concentration in the serum of each patient was measured by the ELISA. The measurement procedure for CXCL11 was as follows. A 1 µg/mL anti-CXCL11 mouse monoclonal antibody solution was added to each well of a 96-well plate and the antibody was sensitized to the well. A serum (100 µL) diluted 5 -fold with a specimen diluent was added to each well and reacted for 2 hours. After washing each well 6 times with the HISCL cleaning solution (300 µL), 100 µL of biotinylated goat anti-human CXCL11 antibody (0.6 µg/mL, 1% BSA, 0.5% casein, PBS, and pH 7.4) was added and reacted for 2 hours. Each well was washed 6 times with the HISCL cleaning solution (300 µL), and then 100 µL of streptavidin ALP was added and reacted for 20 minutes. After washing each well 6 times with the HISCL cleaning solution (300 µL), 100 µL of a mixture of R4 and R5 reagents at a ratio of 1 : 2 was added to each well, and the luminescence signal was counted with a plate reader. As the anti-CXCL11 antibody, Duoset DY672 manufactured by R&D Systems, Inc. was used.

### (3) Measurement results

Table 3 shows the concentrations of CCL17, CCL22, CXCL9, CXCL10, and CXCL11 in the decreased group and the stable group. In the table, the concentrations of CCL17, CCL22, CXCL9, CXCL10, and CXCL11 in each group are shown by median, first and third quartiles. As shown in Table 3, the CCL17 concentration in the serum was significantly higher in the decreased group. Furthermore, the CXCL11 concentration in the serum was significantly higher in the stable group. On the other hand, the serum concentrations of CCL22, CXCL9, and CXCL10 were slightly higher in the stable group, but there was no significant difference between the decreased group and stable group.

**[Table 3]**

| | Decreased group | Stable group | P |
|---|---|---|---|
| CC117 (pg/mL) | 676.1 (569.9 - 916.0) | 411.4 (200.4 - 605.7) | <0.001 |
| CCL22 (pg/mL) | 1270.1 (927.9 - 1478.3) | 1395.0 (1018.9 - 1529.2) | 0.688 |
| CXCL9 (pg/mL) | 15.9 (10.0 - 21.3) | 23.3 (15.4 - 38.5) | 0.068 |
| CXCL10 (pg/mL) | 217.2 (202.3 - 279.9) | 374.3 (218 - 585.3) | 0.144 |
| CXCL11 (pg/mL) | 0.1 (0.0 - 4.4) | 21.2 (1.1 - 34.6) | 0.008 |

### (4) Multivariate analysis by multiple logistic analysis

By multiple logistic analysis, multivariate analysis was performed using age, gender, the CCL17 concentration in the serum, the CXCL9 concentration in the serum, an infiltration shadow, and lymphocyte ratio (BAL lym%) in bronchoalveolar lavage fluid as explanatory variables. SPSS Statisticsversion 22.0 (IBM) was used for the analysis. The results are shown in Table 4. As shown in Table 4, the concentrations of CCL17 and CXCL9 in the serum were independently shown to be significantly associated with the decrease in a respiratory function.

**[Table 4]**

| | P | 95% confidence interval | Odds |
|---|---|---|---|
| COL17 | 0.045 | 0.749 - 0.997 | 0.864 |
| CXCL9 | 0.022 | 1.001 - 1.011 | 1.006 |

### (5) Cluster analysis

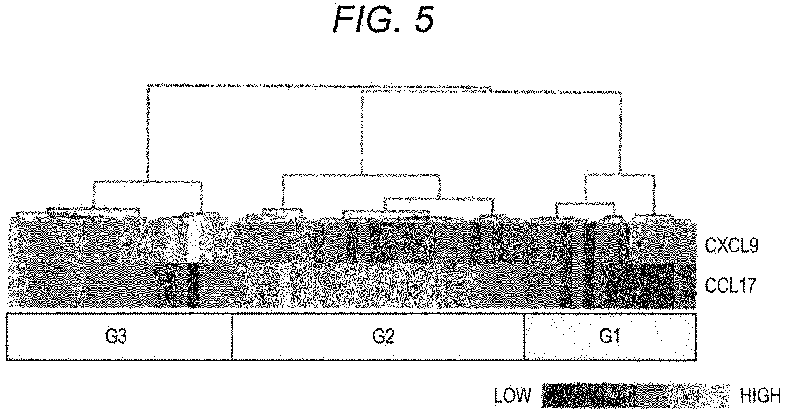

Sixty-one patients were classified by cluster analysis based on the concentrations of CCL17 and CXCL9 in the serum. The cluster analysis was performed by the complete linkage method based on the Euclidean distance. The results are shown in FIG. 5. The analysis revealed that patients with interstitial pneumonia were stratified into three groups of G1, G2 and G3, as shown in Table 5. G1 is a group with a low CCL17 concentration regardless of the CXCL9 concentration, G2 is a group with a low CXCL9 concentration and a high CCL17 concentration, and G3 is a group with a high CXCL9 concentration and a high CCL17 concentration.

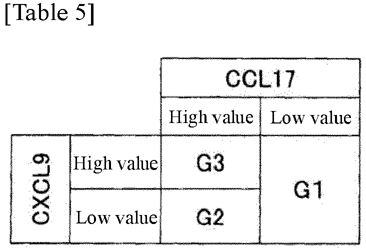

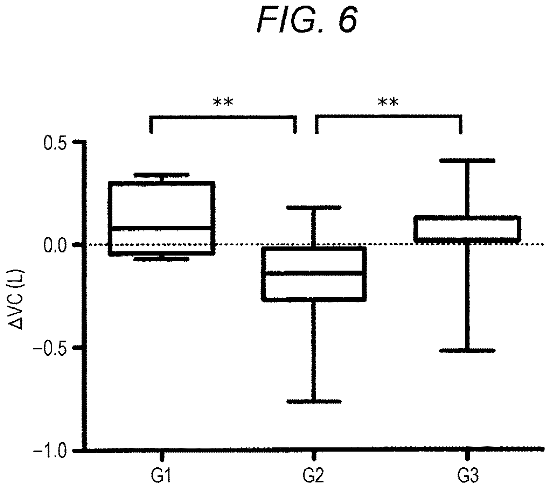

Changes in respiratory function in each group were examined. The results are shown in Table 6 and Fig. 6 In the table, VC (Vital capacity) shows vital capacity, and DL_{CO} (Diffusing capacity of lung for carbon monoxide) shows lung diffusion capacity. In the table, the amount of change in a respiratory function in each group is shown by the median, the first and third quartiles. As shown in Table 6, the vital capacity was decreased by 5% or more in the G2 group. Furthermore, as shown in Fig. 6, the vital capacity decreased significantly in the G2 group compared to the G1 and G3 groups. Thus, it was found that the G2 group had a marked decrease in a respiratory function.

**[Table 6]**

| Group | G1 | G2 | G3 |
|---|---|---|---|
| Number of persons | 15 | 26 | 20 |
| Age | 60.0 | 62.0 | 69.0 |
| Δ VC | 0.08 (-0.04 - 0.30) ^{¶} | -0.14 (-0.27 - -0.02) ^{§} | 0.03 (0.02 - 0.13) |
| Δ %VC | 3.3 (-1.6 - 10.1) ^{¶} | -5.5 (-8.6 - -0.3) ^{§} | 1.4 (0.5 - 5.7) |
| Δ DL_{CO} | 0.41 (-0.85 - 1.09) | -0.36 (-1.74 - 0.58) | -0.30 (-0.47 - 0.54) |
| Δ %DL_{CO} | 3.5 (-4.9 - 5.1) | -1.3 (-8.2 - 2.2) | -2.9 (-4.9 - 3.2) |

| | | | |
|---|---|---|---|
| ¶: *P* < 0.01 versus G2. §: *P* < 0.01 versus G3. | | | |

### (6) Median test

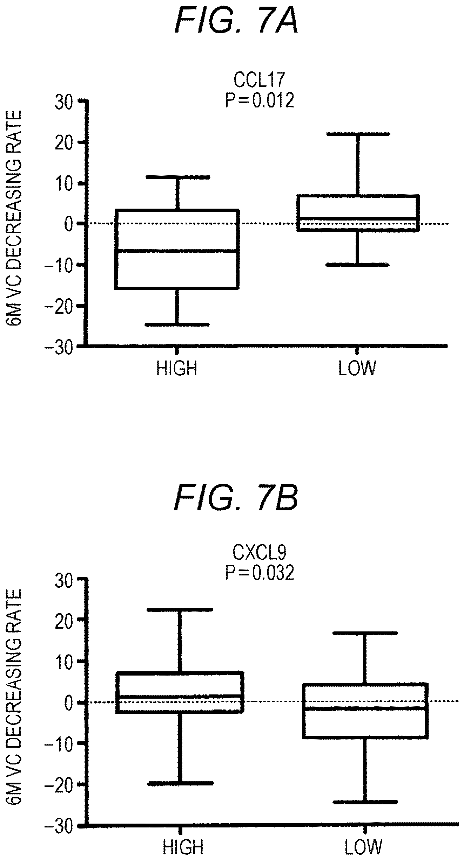

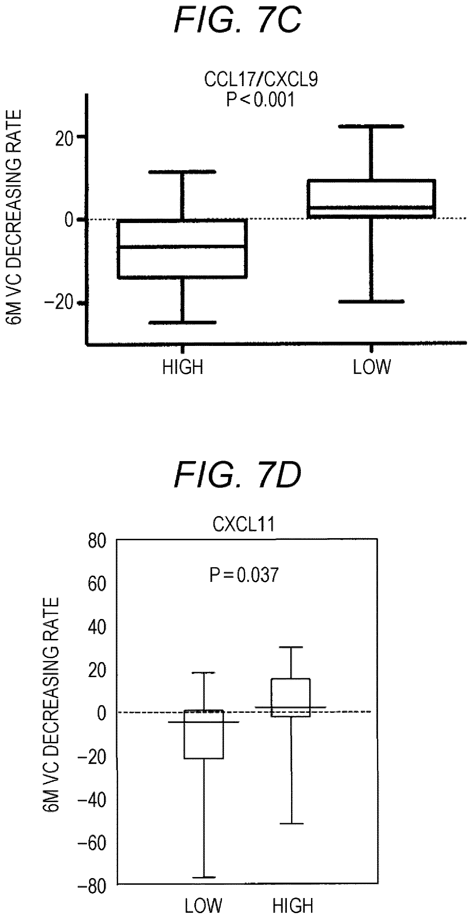

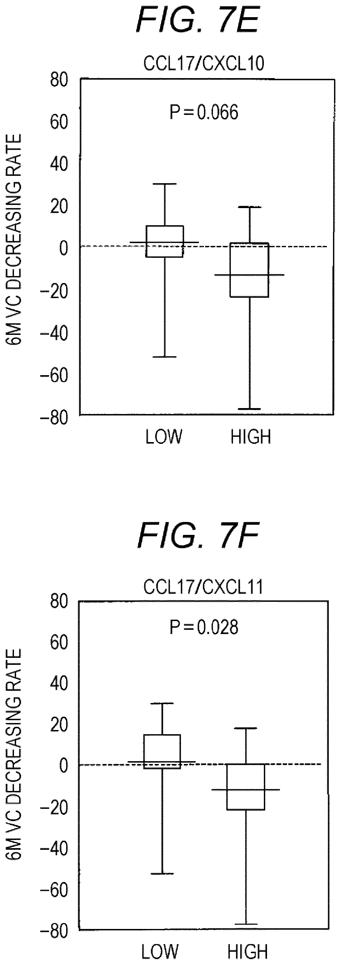

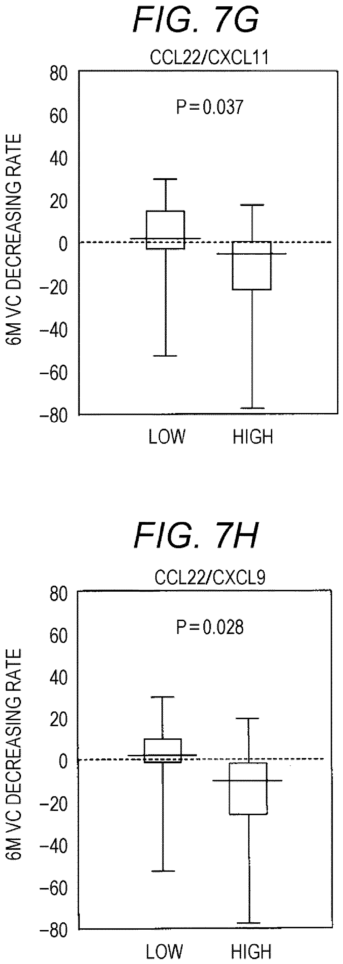

Patients were divided into two groups (high value group and low value group) with the median of a serum CCL17 concentration, a serum CXCL9 concentration, a serum CXCL11 concentration, the ratio of the serum CCL17 concentration to the serum CXCL9 concentration (CCL17/CXCL9), the ratio of the serum CCL17 concentration to a serum CXCL10 concentration (CCL17/CXCL10), the ratio of the serum CCL17 concentration to the serum CXCL11 concentration (referred to as CCL17/CXCL11), the ratio of a serum CCL22 concentration to the serum CXCL11 concentration (CCL22/CXCL11), and the ratio of the serum CCL22 concentration to the serum CXCL9 concentration (CCL22/CXCL9). The decreasing rate (%) of a respiratory function (vital capacity) after 6 months in each group was compared. The results are shown in Figs. 7A to 7H. In these drawings, "High" and "HIGH" are the high value group, and "Low" and "LOW" are the low value group. In these drawings, when the 6M VC decreasing rate is less than 0%, it indicates that the vital capacity after 6 months has decreased, and when the 6M VC decreasing rate is more than 0%, it indicates that the vital capacity after 6 months has increased. As shown in Figs. 7A, 7C, 7F, 7G, and 7H, in the group with the high serum CCL17 concentration, CCL17/CXCL9, CCL17/CXCL11, CCL22/CXCL11, or CCL22/CXCL9, a significant decrease in respiratory function was observed compared with the low value group. As shown in Figs. 7B and 7D, in the group with the low serum CXCL9 concentration or serum CXCL11 concentration, a significant decrease in a respiratory function was observed compared with the high value group. From Fig. 7E, in the group with the high CCL17/CXCL10, a tendency of a decrease in a respiratory function was observed, but there was no significant difference between the high value group and low value group.

### (7) ROC analysis

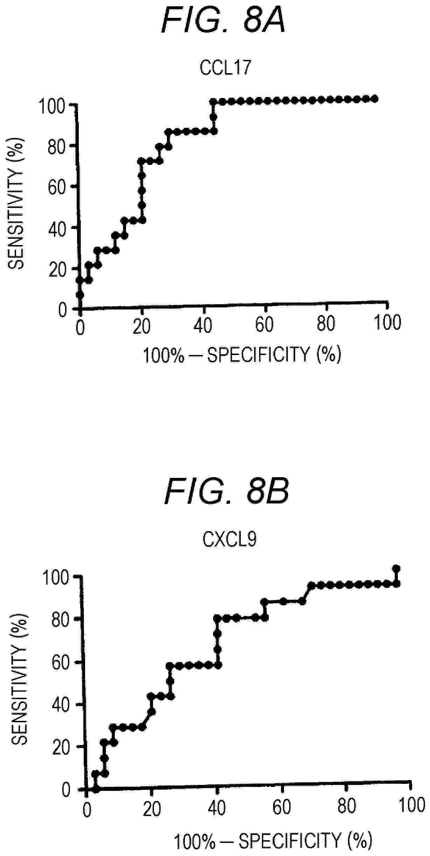

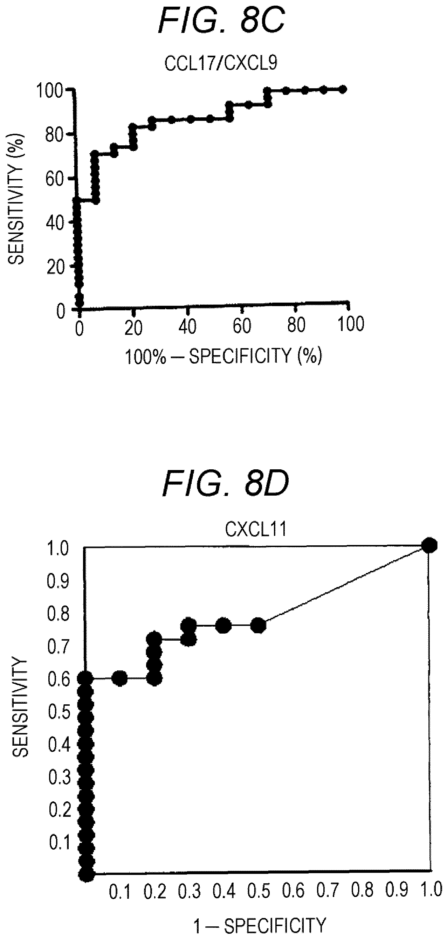

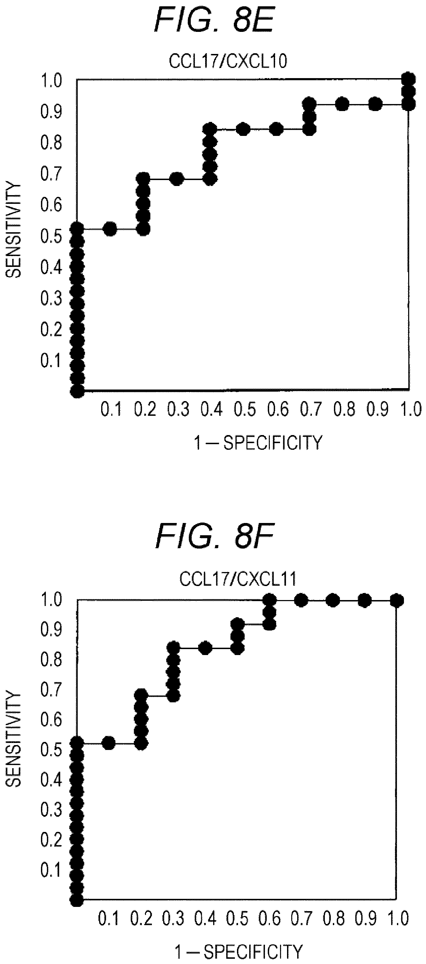

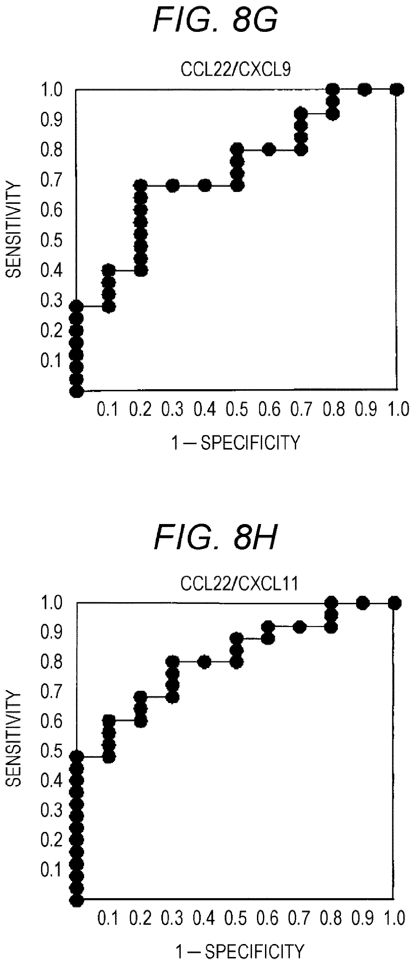

For each value of the serum CCL17 concentration, serum CXCL9 concentration, serum CXCL11 concentration, CCL17/CXCL9, CCL17/CXCL10, CCL17/CXCL11, CCL22/CXCL9, and CCL22/CXCL11, an ROC analysis was used to determine the optimal cutoff value for discriminating between a patient with a decrease in a respiratory function (vital capacity) after 6 months of 5% or more and a patient with the decrease in a respiratory function of less than 5%. The discrimination sensitivity, specificity, and area under the curve (AUC) were calculated based on the set cutoff value. The obtained ROC curves are shown in Figs. 8A to 8H. Table 7 shows the cutoff values of the serum CCL17 concentration, serum CXCL9 concentration, serum CXCL11 concentration, CCL17/CXCL9, CCL17/CXCL10, CCL17/CXCL11, CCL22/CXCL9, and CCL22/CXCL11, respectively, the discrimination sensitivity using the cutoff values, specificity, and AUC.

**[Table 7]**

| Biomarker | Cutoff value | Sensitivity (%) | Specificity (%) | AUC |
|---|---|---|---|---|
| CCL17 | 539.5 | 85.7 | 70.5 | 0.813 |
| CXCL9 | 20.9 | 78.6 | 58.8 | 0.67 |
| CXCL11 | 15.4 | 60 | 100 | 0.784 |
| CCL17/CXCL9 | 24.8 | 70.6 | 92.9 | 0.849 |
| CCL17/CXCL10 | 1.38 | 52 | 100 | 0.768 |
| CCL17/CXCL11 | 1880 | 84 | 70 | 0.832 |
| CCL22/CXCL9 | 52.86 | 68 | 80 | 0.724 |
| CCL22/CXCL11 | 1729 | 80 | 70 | 0.808 |

From Table 7, it was found that it is possible to discriminate between a patient with a decrease in respiratory function (vital capacity) of 5% or more after 6 months and a patient with the decrease of less than 5% according to each value of the serum CCL17 concentration, serum CXCL9 concentration, serum CXCL11 concentration, CCL17/CXCL9, CCL17/CXCL10, CCL17/CXCL11, CCL22/CXCL9, and CCL22/CXCL11. Therefore, CCL17, CXCL9, and CXCL11 were shown to be biomarkers that enable the determination of the risk of a decrease in a respiratory function. It was also shown that the risk of a decrease in a respiratory function can be determined by combining these biomarkers with CXCL10 and CCL22.

### DESCRIPTION OF REFERENCE SIGNS

- 11, 21:: Reagent kit
- 12, 22:: First container
- 13, 23:: Second container
- 14, 24:: Third container
- 15, 25:: Packing box
- 16, 26:: Package leaflet
- 27:: Solid phase (96-well microplate)
- 10:: Determination device
- 20:: Measuring device
- 30:: Computer system

## Claims

1. A method for obtaining information on a risk of a decrease in a respiratory function of a patient with interstitial pneumonia,
the method comprising measuring at least one biomarker in a biological sample of the patient with interstitial pneumonia, wherein the biomarker comprises CCL17 and CXCL9, and a measurement result of the biomarker is an index of the risk of a decrease in the respiratory function of the patient.

2. A method for obtaining information on a risk of a decrease in a respiratory function of a patient with interstitial pneumonia,
the method comprising measuring at least one biomarker in a biological sample of the patient with interstitial pneumonia, wherein the biomarker comprises CCL17, CXCL9, and CXCL11, and a measurement result of the biomarker is an index of the risk of a decrease in the respiratory function of the patient.

3. The method according to claim 1 or 2, wherein the risk of a decrease in the respiratory function is a risk of a decrease in vital capacity 6 months after collection of the biological sample.

4. The method according to claim 3, wherein the risk of a decrease in vital capacity is a risk that the vital capacity 6 months after the collection of the biological sample is decreased by 5% or more from the vital capacity at a time of the collection of the biological sample.

5. The method according to any one of claims 1 to 4, wherein the patient with interstitial pneumonia is an untreated patient.

6. The method according to any one of claims 1 to 5, wherein the patient with interstitial pneumonia is a patient with hypersensitivity pneumonia.

7. The method according to any one of claims 1 to 6, wherein the biological sample is a blood sample.

8. The method according to any one of claims 1 to 7, wherein the measurement result of the biomarker is at least one selected from a group consisting of a measured value of CCL17, a measured value of CXCL9, a ratio of the measured value of CCL17 to the measured value of CXCL9, and a ratio of the measured value of CXCL9 to the measured value of CCL17.

9. The method according to any one of claim 2 and claims 3 to 7 depending on claim 2, wherein the measurement result of the biomarker is at least one selected from a group consisting of a measured value of CCL17, a measured value of CXCL9, a measured value of CXCL11, a ratio of the measured value of CCL17 to the measured value of CXCL9, a ratio of the measured value of CXCL9 to the measured value of CCL17, a ratio of the measured value of CXCL11 to the measured value of CCL17, and a ratio of the measured value of CCL17 to the measured value of CXCL11.

10. The method of any one of claim 1 and claims 3 to 8 depending on claim 1, wherein the biomarker further comprises at least one selected from a group consisting of CCL22, CXCL10, and CXCL11.

11. The method according to any one of claim 2, claims 3 to 7 depending on claim 2, and claim 9, wherein the biomarker further comprises at least one selected from a group consisting of CCL22 and CXCL10.

12. The method according to any one of claims 1 to 11, wherein when the measurement result of the biomarker is a measured value of CCL17 or a ratio of the measured value of CCL17 to a measured value of CXCL9, and the measured value or the value of the ratio is higher than a predetermined threshold corresponding to each value, it is suggested that the risk of a decrease in the respiratory function is high.

13. The method according to any one of claims 1 to 11, wherein when the measurement result of the biomarker is a measured value of CCL17 or a ratio of the measured value of CCL17 to a measured value of CXCL9, and the measured value or the value of the ratio is lower than a predetermined threshold corresponding to each value, it is suggested that the risk of a decrease in the respiratory function is low.

14. The method according to claim 10 or 11, wherein when the measurement result of the biomarker is a measured value of CCL17, a ratio of the measured value of CCL17 to a measured value of CXCL9, a ratio of the measured value of CCL17 to a measured value of CXCL11, a ratio of a measured value of CCL22 to the measured value of CXCL11, or a ratio of the measured value of CCL22 to the measured value of CXCL9, and the measured value or the value of the ratio is higher than a predetermined threshold corresponding to each value, it is suggested that the risk of a decrease in the respiratory function is high.

15. The method according to claim 10 or 11, wherein when the measurement result of the biomarker is a measured value of CCL17, a ratio of the measured value of CCL17 to a measured value of CXCL9, a ratio of the measured value of CCL17 to a measured value of CXCL11, a ratio of a measured value of CCL22 to the measured value of CXCL11, or a ratio of the measured value of CCL22 to the measured value of CXCL9, and the measured value or the value of the ratio is lower than a predetermined threshold corresponding to each value, it is suggested that the risk of a decrease in the respiratory function is low.

16. A method for determining a risk of a decrease in a respiratory function of a patient with interstitial pneumonia,
the method comprising
measuring at least one biomarker in a biological sample of the patient with interstitial pneumonia, and
determining the risk of a decrease in the respiratory function of the patient based on a measurement result of the biomarker,
the biomarker comprising CCL17 and CXCL9.

17. A method for determining a risk of a decrease in a respiratory function of a patient with interstitial pneumonia,
the method comprising
measuring at least one biomarker in a biological sample of the patient with interstitial pneumonia, and
determining the risk of a decrease in the respiratory function of the patient based on a measurement result of the biomarker,
the biomarker comprising CCL17, CXCL9, and CXCL11.

18. The method according to claim 16 or 17, wherein when the measurement result of the biomarker is a measured value of CCL17, a ratio of the measured value of CCL17 to a measured value of CXCL9, or a ratio of the measured value of CCL17 to a measured value of CXCL11, and the measured value or the value of the ratio in the determining is higher than a predetermined threshold corresponding to each value, the risk of a decrease in the respiratory function is determined to be high.

19. The method according to claim 16 or 17, wherein when the measurement result of the biomarker is a measured value of CCL17, a ratio of the measured value of CCL17 to a measured value of CXCL9, or a ratio of the measured value of CCL17 to a measured value of CXCL11, and the measured value or the value of the ratio in the determining is lower than a predetermined threshold corresponding to each value, the risk of a decrease in the respiratory function is determined to be low.

20. The method according to claim 16 or 17, wherein when the measurement result of the biomarker is a measured value of CXCL9, a measured value of CXCL11, a ratio of the measured value of CXCL9 to a measured value of CCL17, or a ratio of the measured value of CXCL11 to the measured value of CCL17, and the measured value or the value of the ratio in the determining is lower than a predetermined threshold corresponding to each value, the risk of a decrease in the respiratory function is determined to be high.

21. The method according to claim 16 or 17, wherein when the measurement result of the biomarker is a measured value of CXCL9, a measured value of CXCL11, a ratio of the measured value of CXCL9 to a measured value of CCL17, or a ratio of a measured value of CXCL11 to the measured value of CCL17, and the measured value or the value of the ratio in the determining is higher than a predetermined threshold corresponding to each value, the risk of a decrease in the respiratory function is determined to be low.

22. The method according to claim 17, wherein
the biomarker further comprises CCL22, and
when the measurement result of the biomarker is a ratio of a measured value of CCL22 to a measured value of CXCL11 or a ratio of the measured value of CCL22 to a measured value of CXCL9, and the measured value or the value of the ratio in the determining is higher than a predetermined threshold corresponding to each value, the risk of a decrease in the respiratory function is determined to be high.

23. The method according to claim 17, wherein
the biomarker further comprises CCL22, and
when the measurement result of the biomarker is a ratio of a measured value of CCL22 to a measured value of CXCL11 or a ratio of the measured value of CCL22 to a measured value of CXCL9, and the measured value or the value of the ratio in the determining is lower than a predetermined threshold corresponding to each value, the risk of a decrease in the respiratory function is determined to be low.

24. The method according to claim 17, wherein
the biomarker further comprises CCL22, and
when the measurement result of the biomarker is a ratio of a measured value of CCL22 to a measured value of CXCL11 or a ratio of the measured value of CCL22 to a measured value of CXCL9, and the measured value or the value of the ratio in the determining is lower than a predetermined threshold corresponding to each value, the risk of a decrease in the respiratory function is determined to be high.

25. The method according to claim 17, wherein
the biomarker further comprises CCL22, and
when the measurement result of the biomarker is a ratio of a measured value of CCL22 to a measured value of CXCL11 or a ratio of the measured value of CCL22 to a measured value of CXCL9, and the measured value or the value of the ratio in the determining is higher than a predetermined threshold corresponding to each value, the risk of a decrease in the respiratory function is determined to be low.

26. A reagent kit for use in the method according to any one of claims 1 to 25, the reagent kit comprising a reagent comprising a substance that can specifically bind to CCL17 and/or a reagent comprising a substance that can specifically bind to CXCL9.

27. A reagent kit for use in the method according to any one of claims 1 to 25, the reagent kit comprising a reagent comprising a substance that can specifically bind to CCL17 and/or a reagent comprising a substance that can specifically bind to CXCL9 and/or a reagent comprising a substance that can specifically bind to CXCL11.

28. A device for determining a risk of a decrease in a respiratory function of a patient with interstitial pneumonia,
the device comprising a computer comprising a processor and a memory under a control of the processor,
wherein the memory records a computer program for the computer to execute:
obtaining a measurement result of at least one biomarker in a biological sample of the patient with interstitial pneumonia; and
determining the risk of a decrease in the respiratory function of the patient based on the measurement result of the biomarker,
the biomarker comprises CCL17 and CXCL9.

29. A device for determining a risk of a decrease in a respiratory function of a patient with interstitial pneumonia,
the device comprising a computer comprising a processor and a memory under a control of the processor,
wherein the memory records a computer program for the computer to execute:
obtaining a measurement result of at least one biomarker in a biological sample of the patient with interstitial pneumonia; and
determining the risk of a decrease in the respiratory function of the patient based on the measurement result of the biomarker,
the biomarker comprises CCL17, CXCL9, and CXCL11.

30. A computer program for determining a risk of a decrease in a respiratory function of a patient with interstitial pneumonia,
wherein the computer program is recorded on a medium that can be read by a computer and is recorded for the computer to execute:
obtaining a measurement result of at least one biomarker in a biological sample of the patient with interstitial pneumonia; and
determining the risk of a decrease in the respiratory function of the patient based on the measurement result of the biomarker,
the biomarker comprises CCL17 and CXCL9.

31. A computer program for determining a risk of a decrease in a respiratory function of a patient with interstitial pneumonia,
wherein the computer program is recorded on a medium that can be read by a computer and is recorded for the computer to execute:
obtaining a measurement result of at least one biomarker in a biological sample of the patient with interstitial pneumonia; and
determining the risk of a decrease in the respiratory function of the patient based on the measurement result of the biomarker,
the biomarker comprises CCL17, CXCL9, and CXCL11.
